(19) Europäisches Patentamt
European Patent Office
Office européen des brevets

(11) EP 1 761 278 B1

(12) **EUROPEAN PATENT SPECIFICATION**

(45) Date of publication and mention of the grant of the patent:
**31.12.2008 Bulletin 2009/01**

(21) Application number: **05747242.5**

(22) Date of filing: **25.04.2005**

(51) Int Cl.:
***A61K 39/39*** *(2006.01)*

(86) International application number:
**PCT/IB2005/001485**

(87) International publication number:
**WO 2005/102385 (03.11.2005 Gazette 2005/44)**

# (54) ADJUVANT COMPOSITION AND METHODS FOR ITS USE

ADJUVANTE ZUSAMMENSETZUNG UND ANWENDUNGSVERFAHREN DAFÜR

COMPOSITION ADJUVANTE ET METHODES D'UTILISATION CORRESPONDANTES

(84) Designated Contracting States:
**AT BE BG CH CY CZ DE DK EE ES FI FR GB GR HU IE IS IT LI LT LU MC NL PL PT RO SE SI SK TR**

(30) Priority: **26.04.2004 US 564959 P**
**18.11.2004 US 629069 P**

(43) Date of publication of application:
**14.03.2007 Bulletin 2007/11**

(73) Proprietor: **Innate Pharma**
**13009 Marseille (FR)**

(72) Inventor: **TIOLLIER, Jérôme**
**F-13008 Marseille (FR)**

(74) Representative: **Gallois, Valérie et al**
**Cabinet BECKER & ASSOCIES**
**25, rue Louis Le Grand**
**75002 Paris (FR)**

(56) References cited:
**WO-A-00/12516**
**WO-A-00/12519**
**WO-A-03/009855**
**WO-A-03/038072**
**WO-A-03/050128**
**WO-A-20/05021708**
**WO-A-20/05054258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 761 278 B1

**Description**

## FIELD OF THE INVENTION

The present invention is directed to a novel vaccine adjuvant which improves the vaccine potency. More specifically, the present invention is directed to the use of a γδT lymphocyte activator as vaccine adjuvant to promote and enhance antigen specific immunological responses, as well as a vaccine composition comprising a γδT lymphocyte activator.

## BACKGROUND OF THE INVENTION

*Preclinical Evidence Using Gamma Delta T Cell Activators*

In recent years, a novel lymphoid lineage, γδ T cells, distinct from mainstream T cells, B cells and NK cells, has been identified. Most human adults share a major γδ T cell subset bearing a particular combination of TCR variable regions (Vγ9 and Vδ2), which represents from 60 to 95 % of peripheral blood γδ T cells and up to 1/20 of total T cells in this site (Bottino et al., J Exp Med. 1988 Aug 1;168(2):491-505, Davodeau et al., Science. 1993 Jun 18;260(5115): 1800-2). Vγ9Vδ2 T cells and NK cells share several phenotypic features (*e.g.* frequent expression of so-called Natural Killer receptors) as well as effector properties (NK-like cytolytic activity, release of pro-inflammatory cytokines), which suggest similar physiological roles played by these two lymphoid subsets. *In vivo* Vγ9Vδ2 T cells are expanded in various physiopathological situations, particularly during (i) infectious processes involving intracellular bacteria (tuberculosis, malaria, tularemia, etc.) and parasites (Ito et al., Chest. 1992;102(1):195-7, Modlin et al., Nature. 1989; 339(6225): 544-8), (ii) autoimmune diseases (for a review see Wen and Hayday, Immunol Res. 1997;16(3):229-41. ; and Hayday and Geng, Curr Opin Immunol. 1997; 9(6):884-9. and (iii) several cancers. Besides, Vγ9Vδ2 T cell clones display *in vitro* both anti-tumor and antibacterial reactivity. In particular they lyse various tumor cell lines (Kobayashi et al., Cancer Immunol Immunother. 2001; 50(3):115-24. These observations suggest an important role for Vγ9Vδ2 T cells in tumor surveillance. Vγγ9Vδ2 T cells have also been reported to be involved in the maturation of dendritic cells (DC). Activated γδ T cells induced the production of IL-12 (p40) and IL-12 (p70) by DC, an effect that involved IFN-gamma production, suggesting that gamma delta T cell activation might result in DC maturation and thereby in enhanced alpha beta T cell responses to infection by microorganisms (Ismaili et al., Clin Immunol. 2002;103(3 Pt 1):296-302).

Vγ9Vδ2 T cells are also activated by small non-peptidic phosphorylated antigens (referred to as "phosphoantigens") that are produced by a wide range of bacteria and intracellular parasites (Constant 1994). These compounds and their derivatives (phosphohalohydrins, PHD, described in PCT Publication No. WO 04/050096 and also in European Patent Publication no. EP B1 109 817) represent the first immunotherapeutic molecule directed to the specific modulation of Vγ9Vδ2 T cells in development and are intended for the treatment of cancer. More recently, since the isolation of (E)-4-Hydroxy-3-methylbut-2-enyl diphosphate (HDMAPP) from *E. coli* cells deficient in the lytB component of the non-mevalonale (MEP) pathway described in Hintz et al. ((2001). FEBS Lett. 509(2): 317-22), other compounds having *in vitro* γδ T cell-modulating activity have been available. A further range of phosphonate compounds reportedly capable of modulating γδ T cell- activity are described in PCT publication no. WO 03/050128. WO 03/009855 <u>discloses the compounds HDMAPP and CHDMAPP, optionally combined in a pharmaceutical composition with immunognes, and mentions their possible use to strength the immune response.</u>

The above observations, suggest an involvement of Vγ9Vδ2 T cells in protective immunity against infectious agents. However, to date there have been no effective means for or attempts to apply these observations to the treatment or prevention of disease in vaccine-based approaches.

*Vaccine Adjuvants*

Vaccines have proven to be successful, highly acceptable methods for the prevention of infectious diseases. They are cost effective, and do not induce antibiotic resistance to the target pathogen or affect normal flora present in the host. In many cases, such as when inducing anti-viral immunity, vaccines can prevent a disease for which there are no viable curative or ameliorative treatments available.

Vaccines function by triggering the immune system to mount a response to an agent, or antigen, typically an infectious organism or a portion thereof that is introduced into the body in a non-infectious or non-pathogenic form. Once the immune system has been "primed" or sensitized to the organism, later exposure of the immune system to this organism as an infectious pathogen results in a rapid and robust immune response that destroys the pathogen before it can multiply and infect enough cells in the host organism to cause disease symptoms.

The agent, or antigen, used to prime the immune system can be the entire organism in a less infectious state, known as an attenuated organism, or in some cases, components of the organism such as carbohydrates, proteins or

peptides representing various structural components of the organism.

In many cases, it is necessary to enhance the immune response to the antigens present in a vaccine in order to stimulate the immune system to a sufficient extent to make a vaccine effective, *i.e.*, to confer immunity. Many protein and most peptide and carbohydrate antigens, administered alone, do not elicit a sufficient antibody response to confer immunity. Such antigens need to be presented to the immune system in such a way that they will be recognized as foreign and will elicit an immune response. To this end, additives (adjuvants) have been devised which immobilize antigens and stimulate the immune response.

Adjuvants can be found in a group of structurally heterogeneous compounds (Gupta et al., 1993, Vaccine, 1 1:293-306). Classically recognized examples of adjuvants include oil emulsions (*e.g.*, Freund's adjuvant), saponins, aluminum or calcium salts (*e.g.*, alum), nonionic block polymer surfactants, lipopolysaccharides (LPS), mycobacteria, tetanus toxoid, and many others. Theoretically, each molecule or substance that is able to favor or amplify a particular situation in the cascade of immunological events, ultimately leading to a more pronounced immunological response can be defined as an adjuvant.

In principle, through the use of adjuvants in vaccine formulations, one can (1) direct and optimize immune responses that are appropriate or desirable for the vaccine; (2) enable mucosal delivery of vaccines, *i.e.*, administration that results in contact of the vaccine with a mucosal surface such as buccal or gastric or lung epithelium and the associated lymphoid tissue; (3) promote cell-mediated immune responses; (4) enhance the immunogenicity of weaker immunogens, such as highly purified or recombinant antigens; (5) reduce the amount of antigen or the frequency of immunization required to provide protective immunity; and (6) improve the efficacy of vaccines in individuals with reduced or weakened immune responses, such as newborns, the aged, and immuno-compromised vaccine recipients.

Although little is known about their mode of action, it is currently believed that adjuvants augment immune responses by any of several means. Some assist in the presentation of antigen to antigen processing cells (APC). Oil-in-water emulsions, water-in-oil emulsions, liposomes and microbeads each assist in presenting antigen to APC. Small antigens or haptens are often linked to larger, immunogenic proteins or polysaccharides to facilitate recognition by the APC. Certain adjuvants have a depot effect holding antigen in place until the body has an opportunity to mount an immune response. Adjuvants may also increase the biological or immunologic half-life of antigens, mimic microbial structures leading to improved recognition of microbially-derived antigens by the pathogen-recognition receptors (PRRs) which are localized on accessory cells from the innate immune system, mimic danger-inducing signals from stressed or damaged cells which serve to initiate an immune response, induce the production of immunomodulatory cytokines or bias the immune response cowards a specific subset of the immune system (*e.g.*, generating Thl- or Th2-polarized response). Mechanisms of adjuvant action are reviewed in PCT publication no. WO 03/009812.

Recent observations strongly suggest that endogenously produced cytokines act as essential communication signals elicited by traditional adjuvants. The redundancy of the cytokine network makes it difficult to ascribe the activity of a particular adjuvant to one or more cytokines. Cytokines crucial for immunogenicity may include the proinflammatory (Type 1) substances: interferon (IFN), tumor necrosis factor (TNF)-alpha, interleukin (IL)-1, IL-6, IL-12, IL- 15 and IL-18, which influence antigen presentation. Others may act more downstream during clonal expansion and differentiation of T and B cells, with IL-2, IL-4 and IFN-gamma as prototypes (Brewer et al., 1996, Eur. J. Immunol., 26:2062-2066; Smith et al., 1998, Immunology, 93:556562). Adjuvants that enhance immune responses through the induction of IFN-, y and delayed type hypersensitivity also elicit the production of IgG subclasses that are the most active in complement-mediated lysis and in antibody-dependent cell-mediated-cytotoxicity effector mechanisms (*e.g.*, IgG2a in mice and IgGl in humans) (Allison, Dev. Biol. Stand., 1998, 92:311; Unkeless, Annu. Rev. Immunol., 1988, 6:251-81; Phillips et al., Vaccine, 1992, 10:151-8).

Adjuvants may perform more than one function. As different adjuvants may have diverse mechanisms of action, their being chosen for use with a particular vaccine may be based on the route of administration to be employed, the type of immune responses desired (*e.g.*, autibody-mediated, cell-mediated, mucosal, etc.), and the particular inadequacy of the primary antigen.

The benefit of incorporating adjuvants into vaccine formulations to enhance immunogenicity must be weighed against the risk that these agents will induce adverse local and/or systemic reactions. Local adverse reactions include local inflammation at the injection site and, rarely, the induction of granuloma or sterile abscess formation. Systemic reactions to adjuvant observed in laboratory animals include malaise, fever, adjuvant arthritis, and anterior uveitis (Allison et al., Mol. Immunol., 1991, 28:279-84; Waters et al., Infect Immun, 1986, 51:816-25). Such reactions often are caused by the interaction of the adjuvant and the antigen itself, or may be due to the type of response to a particular antigen the adjuvant produces, or the cytokine profile the adjuvant induces. Thus, many potent immuno-adjuvants, such as Freund's Complete or Freund's Incomplete Adjuvant, are toxic and are therefore useful only for animal research purposes, not human vaccinations. Due to the limited range of immuno-adjuvants available there is therefore a need in the art for new and improved immuno-adjuvants.

## SUMMARY OF THE INVENTION

Observations made by the present inventors have led them to a hypothesis that gamma delta T cell-responses induced by the compounds of Formula I may contribute to enhance or augment immune responses involved in combating various infections and forms of cancer. The present inventors and co-workers have recently observed that the administration of HDMAPP to cynomolgus monkeys resulted rapidly in strong activation of γδ T cells at very low doses suitable for use in a vaccine adjuvant application. Moreover, by observing re-stimulation of γδ T cells, the inventors and co-workers provide that the compounds can be administered repeatedly so as to produce consistent immune enhancing or immune augmenting effect. The latter is important insofar as many vaccines require more than one vaccine dose, for example requiring a priming and boosting dose, or a plurality of boosting doses. (See co-pending PCT publication No. WO 04/050096). Importantly, in addition to its ability to stimulate immune responses, the inventors have demonstrated that the compounds of Formula I do not induce toxicity in rodents and monkeys. Finally, unlike many other newly developed adjuvants (see below), HDMAPP and related the compounds of Formula I can be produced synthetically with reasonable yields and efficiency as shown in the Examples. All of these factors, in combination with an ability to stimulate a CTL or humoral immune response, make the compounds of Formula I desirable adjuvants.

Accordingly, the present invention discloses methods and compositions for enhancing and/or augmenting the immune response against an antigen in a mammal, notably a human, involving the conjoint immunization of the mammal with (i) a composition comprising an antigen and (ii) an adjuvant comprising a compound of Formula I. Preferably said composition comprising an antigen comprises a killed, inactivated or attenuated pathogen, microorganism or parasite. In other aspect, said composition comprising an antigen preferably comprises an enriched or purified polypeptide, lipid, polysaccharide, glycoprotein, glycolipid or nucleic acid antigen. Preferably said composition comprises at least 1, 2, 3, 4, 5, 10 or 15 distinct antigens, for example at least 1, 2, 3, 4, 5, 10 or 15 distinct polypeptides, or nucleic acids encoding such polypeptides.

The adjuvant composition will comprise an effective amount of a γδ T cell activator of Formula I, preferably of Formula 11 or III, said amount being an effective amount allowing the elicitation a cytotoxic T lymphocyte (CTL) response, or elicitation of both a humoral response and a CTL response of the adjuvant composition with respect to at least one antigen. Preferably the γδ T cell activator of Formula I, preferably of Formula 11 or III, is present in an amount effective to produce a greater immunological effect in eliciting cytotoxic T lymphocyte (CTL) response or both a humoral response and a CTL response when administered conjointly with an antigen than that immunological effect produced when said antigen is administered in the absence of the adjuvant.

Adjuvant-mediated enhancement and/or extension of the duration of the immune response can be assessed by any method known in the art including without limitation one or more of the following: (i) an increase in the number of antibodies produced in response to immunization with the adjuvant/antigen combination versus those produced in response to immunization with the antigen alone; (ii) an increase in the number of T cells recognizing the antigen or the adjuvant; and (iii) an increase in the level of one or more Type I cytokines.

The antigen component of the composition can be selected from virtually any antigen, antigenic determinant or hapten of medical or veterinary interest, and particularly for those antigens for which an increase in immunogenicity is desired.

Therefore, the present invention concerns the use of a γδ T cell activator of Formula I, preferably of Formula II or III, more preferably HDMAPP or CHDMAPP, as a vaccine adjuvant. The present invention further concerns a vaccine composition comprising an antigen or a combination of antigens, and a γδ T cell activator of Formula I, preferably of Formula II or III, more preferably HDMAPP or CHDMAPP. Preferably, said composition comprises a therapeutically effective amount of antigen and an immune response enhancing or immune response augmenting amount of a γδ T cell activator. Preferably, said vaccine composition prevents a microbial infection. Said microbial infection is caused by a microbe selected from the group consisting of viruses, fungi, parasites, yeast, bacteria, and protozoa. In a particular embodiment, said vaccine composition is BCG vaccine composition. Alternatively, said vaccine composition prevents or is a treatment against a tumor.

The present invention further concerns a vaccine kit comprising a suitable container containing a vaccine composition according to the present invention, more particularly comprising an antigen or a combination of antigens, and a γδ T cell activator of Formula I, preferably of Formula 11 or III, more preferably HDMAPP or CHDMAPP. Optionally, said vaccine can comprise two separate suitable containers, one containing the antigen or the combination of antigens and the other containing a γδ T cell activator of Formula I, preferably of Formula II or III, more preferably HDMAPP or CHDMAPP. Optionally, said container can be a syringe. Alternatively, said vaccine kit comprises one or two containers and a syringe.

The present invention concerns a method of improving the potency of a vaccine in a subject, or of immunizing a subject against a disease, more particularly a microbial infection, comprising the steps of : administering to said subject a composition comprising an antigen or a combination of antigens; and, conjointly administering to said subject a γδ T cell activator of Formula I, preferably of Formula II or III, more preferably HDMAPP or CHDMAPP, more particularly an

immune response enhancing amount thereof. Preferably, the γδ T cell activator, when administered conjointly with a composition comprising an antigen, is administered in an amount sufficient to enhance an immune response over that observed with said composition comprising an antigen in the absence of the γδ T cell activator. Preferably said composition comprising an antigen comprises a killed, inactivated or attenuated pathogen, microorganism or parasite. In other aspect, said composition comprising an antigen preferably comprises an enriched or purified polypeptide, lipid, polysaccharide, glycoprotein, glycolipid or nucleic acid antigen.

The present invention also concerns a method of immunizing a subject against a disease, more particularly a microbial infection, in a subject comprising administering to said subject (i) a composition comprising an antigen, and (ii) a γδ T cell activator of Formula I, preferably of Formula II or III, more preferably HDMAPP or CHDMAPP. Preferably the γδ T cell activator is administered in an immune response enhancing amount. Preferably the γδ T cell activator and the composition comprising an antigen are administered as a single vaccine composition in a therapeutically effective amount.

Preferably, said γδ T cell activator is together with a pharmaceutically acceptable carrier. In a first aspect, said administrations of said antigen or combination of antigens and said γδ T cell activator are simultaneously. In a second aspect, said administrations of said antigen or combination of antigens and said γδ T cell activator are sequentially. More particularly, said γδ T cell activator can be administered prior to, concurrently with or subsequent to administration of an antigen or a combination of antigens to a subject for immunization purposes. Preferably, said antigen or combination of antigens are microbial antigens, preferably, viral, bacterial, fungal, protozoan, yeast or parasite antigens. In a preferred embodiment, said antigen is an antigen of *Mycobacterium bovis*. Optionally, said antigen or combination of antigens is a tumoral antigen.

## DESCRIPTION OF THE FIGURES

Figure 1 shows the synthesis scheme for the compound referred to herein as CHDMAPP, the complete synthesis of which is described in Example 2.

Figure 2 shows *in vitro* biological activity of γδ T cells treated with test compounds HDMAPP and CHDMAPP, as assessed using a TNFα release assay. For purposes of comparison, the compounds isopentenyl pyrophosphate (IPP) and bromohyrdin pyrophosphate (BrHPP or Phosphostim) were included in the *in vivo* comparison. The *in vitro* EC50 for HDMAPP was determined to be about 0.758 nM while the *in vitro* EC50 for CDMAPP is about 0.873 nM. By contrast, the less potent BrHPP and IPP showed an EC50 value of about 18.59 nM and 30.98 μM, respectively.

Figure 3 shows the *in vivo* response of 16 monkeys to BCG vaccination with and without a phosphoantigen adjuvant. Monkey were divided into two groups of 8 individuals (Phosphoantigen + Hybrid-I vaccine compared to Hybrid-I vaccine alone) and biological response was determined by assessing serum dosage/level of various cytokines known to act as key mediators of lymphoctye recruitment, proliferation and/or activation, including IFNγ, TNF-alpha, IL2, IL4, IL5 and IL6. Addition of the Phosphoantigen compound to the Hybrid-I formulation produced a large and statistically significant increase in cytokine production over that observed with the Hybrid-I formulation alone.

## DETAILED DESCRIPTION

Immune systems are classified into two general systems, the "innate" or "natural" immune system and the "acquired" or "adaptive" immune system. It is thought that the innate immune system initially keeps the infection under control, allowing time for the adaptive immune system to develop an appropriate response. Recent studies have suggested that the various components of the innate immune system trigger and augment the components of the adaptive immune system, including antigen-specific B and T lymphocytes (Fearon and Locksley, *supra*; Kos, 1998, Immunol. Res., 17: 303; Romagnani, 1992, Immunol. Today, 13: 379; Banchereau and Steinman, 1988, Nature, 392: 245). The term "innate immunity" or "natural immunity" refers to innate immune responses that are not affected by prior contact with the antigen. Cells of the innate immune system, including macrophages and dendritic cells (DC), take up foreign antigens through pattern recognition receptors, combine peptide fragments of these antigens with MHC class I and class II molecules, and stimulate naive $CD8^+$ and $CD4^+$ T cells respectively (Banchereau and Steinman, *supra;* Holmskov et al., 1994, Immunol. Today, 15: 67; Ulevitch and Tobias, 1995, Annu. Rev. Immunol., 13: 437). Professional antigen-presenting cells (APC) communicate with these T cells leading to the differentiation of naive $CD4^+$ T cells into T-helper 1 (Thl) or T-helper 2 (Th2) lymphocytes that mediate cellular and humoral immunity, respectively (Trinchieri, 1995, Annu. Rev. Immunol., 13: 251; Howard and O'Garra, 1992, Immunol. Today, 13: 198; Abbas et al., 1996, Nature, 383: 787; Okamura et al., 1998, Adv. Immunol., 70: 281; Mosmann and Sad, 1996, Immunol. Today, 17: 138; O'Garra, 1998,

Immunity, 8: 275.

The term "acquired immunity" or "adaptive immunity" is used herein to mean active or passive, humoral or cellular immunity that is established during the life of an animal, is specific for the inducing antigen, and is marked by an enhanced response on repeated encounters with said antigen. A key feature of the T lymphocytes of the adaptive immune system is their ability to detect minute concentrations of pathogen-derived peptides presented by MHC molecules on the cell surface.

Protective immunity induced by vaccination is dependent on the capacity of the vaccine to elicit the appropriate immune response to resist or eliminate the pathogen. Depending on the pathogen, this may require a cell-mediated and/or humoral immune response. It is often desirable to enhance the immunogenic potency of an antigen in order to obtain a stronger immune response in the organism being immunized, orient the immune response toward a particular type of response, and strengthen host resistance to the antigen-bearing agent. A substance that enhances the immunogenicity of an antigen with which it is administered is known as an adjuvant.

Within the meaning of the present invention, the term "conjoint administration" is used to refer to administration of an immune adjuvant and an antigen simultaneously in one composition, or simultaneously in different compositions, or sequentially. For the sequential administration to be considered "conjoint", however, the antigen and adjuvant must be administered separated by a time interval that still permits the adjuvant to augment the immune response to the antigen. For example, when the antigen is a polypeptide, the antigen and adjuvant are administered on the same day (*e.g.* within 24 hours of one another), preferably within an hour of each other, and most preferably simultaneously. However, when nucleic acid is delivered to the subject and the polypeptide antigen is expressed in the subject's cells, the adjuvant is administered within 24 hours of nucleic acid administration, preferably within 6 hours.

As used herein, the term "immunogenic" means that an agent is capable of eliciting a humoral or cellular immune response, and preferably both. An immunogenic entity is also antigenic. An immunogenic composition is a composition that elicits a humoral or cellular immune response, or both, when administered to an animal having an immune system.

The term "antigen" refers to any agent (*e.g.*, protein, peptide, lipid, polysaccharide, glycoprotein, glycolipid, nucleic acid or any combination of any of the foregoing) that, when introduced into a host, animal or human, having an immune system (directly or upon expression as in, *e.g.*, DNA vaccines), is recognized by the immune system of the host and is capable of eliciting an immune response. As defined herein, the antigen-induced immune response can be humoral or cell-mediated, or both. An agent is termed "antigenic" when it is capable of or comprises a component capable of specifically interacting with an antigen recognition molecule of the immune system, such as an immunoglobulin (antibody) or T cell antigen receptor (TCR). Examples of preferred antigens are "surface antigens", *i.e.*, expressed naturally on the surface of a pathogen, or the surface of an infected cell, or the surface of a tumor cell. A molecule that is antigenic need not be itself immunogenic, *i.e.*, capable of eliciting an immune response without an adjuvant or carrier. An antigen may be "species-specific", referring to an antigen that is only present in or derived from a particular species.

The term "epitope" or "antigenic determinant" refers to any portion of an antigen recognized either by B cells, or T cells, or both. Preferably, interaction of such epitope with an antigen recognition site of an immunoglobulin (antibody) or T cell antigen receptor (TCR) leads to the induction of antigen-specific immune response. T cells recognize proteins only when they have been cleaved into smaller peptides and are presented in a complex called the "major histocompatability complex (MHC)" located on another cell's surface. There are two classes of MHC complexes-class I and class II, and each class is made up of many different alleles. Class I MHC complexes are found on virtually every cell and present peptides from proteins produced inside the cell. Thus, class I MMC complexes are useful for killing cells infected by viruses or cells which have become cancerous as the result of expression of an oncogene. T cells which have a protein called CD8 on their surface, bind specifically to the MHC class I/peptide complexes via the T cell receptor (TCR). This leads to cytolytic effector activities. Class II MHC complexes are found only on antigen-presenting cells (APC) and are used to present peptides from circulating pathogens which have been endocytosed by APCs. T cells which have a protein called CD4 bind to the MHC class II/peptide complexes via TCR. This leads to the synthesis of specific cytokines which stimulate an immune response. To be effectively recognized by the immune system via MHC class I presentation, an antigenic polypeptide has to contain an epitope of at least about 8 to 10 amino acids, while to be effectively recognized by the immune system via MHC class II presentation, an antigenic polypeptide has to contain an epitope of at least about 13 to 25 amino acids. See, *e.g.,* Fundamental Immunology, 3rd Edition, W. E. Paul ed., 1999, Lippincott-Raven Publ.

A "vaccine" is an immunogenic or antigenic composition that can be used to elicit protective immunity or a protective immune response in a recipient. The protective immunity may be towards pathogens such as viruses, fungi, parasites, yeast, bacteria, and protozoa. More particularly, said composition is capable of eliciting protection against infections, whether partial or complete. A vaccine may also be useful for treatment of an individual, in which case it is called a therapeutic vaccine. Said vaccine compositions may include prophylactic as well as therapeutic vaccine compositions. The term "DNA vaccine" is an informal term of art, and is used herein to refer to a vaccine delivered by means of a recombinant vector. An alternative, and more descriptive term used herein is "vector vaccine" (since some potential vectors, such as retroviruses and lentiviruses are RNA viruses, and since in some instances non-viral RNA instead of DNA is delivered to cells through the vector). Generally, the vector is administered *in vivo,* but ex vivo transduction of

appropriate antigen presenting cells, such as dendritic cells (DC), with administration of the transduced cells *in vivo,* is also contemplated. The term "treat" is used herein to mean to relieve or alleviate at least one symptom of a disease in a subject. Within the meaning of the present invention, the term "treat" may also mean to prolong the prepatency, *i.e.*, the period between infection and clinical manifestation of a disease. Preferably, the disease is either infectious disease (*e.g.*, viral, bacterial, parasitic, or fungal) or malignancy (*e.g.*, solid or blood tumors such as sarcomas, carcinomas, gliomas, blastomas, pancreatic cancer, breast cancer, ovarian cancer, prostate cancer, lymphoma, leukemia, melanoma, etc.).

The term "protect" is used herein to mean prevent or treat, or both, as appropriate, development or continuance of a disease in a subject. Within the meaning of the present invention, the disease is selected from the group consisting of infection (*e.g.*, viral, bacterial, parasitic, or fungal) and malignancy (*e.g.*, solid or blood tumors such as sarcomas, carcinomas, gliomas, blastomas, pancreatic cancer, breast cancer, ovarian cancer, prostate cancer, lymphoma, leukemia, melanoma, etc.). For example, a therapeutic administration of a tumor-specific antigen conjointly with an adjuvant comprising an HDMAPP compound or another compound of Formula I can enhance an anti-tumor immune response leading to slow-down in tumor growth and metastasis or even tumor regression.

The term "protective immunity" refers to an immune response in a host animal (either active/acquired or passive/innate, or both) which leads to inactivation and/or reduction in the load of said antigen and to generation of long-lasting immunity (that is acquired, *e.g.*, through production of antibodies), which prevents or delays the development of a disease upon repeated exposure to the same or a related antigen. A "protective immune response" comprises a humoral (antibody) immunity or cellular immunity, or both, effective to, *e.g.*, eliminate or reduce the load of a pathogen or infected cell (or produce any other measurable alleviation of the infection), or to reduce a tumor burden in an immunized (vaccinated) subject. Within the meaning of the present invention, protective immunity may be partial.

As used herein, the term "augment the immune response" means enhancing or extending the duration of the immune response, or both. When referred to a property of an agent (*e.g.*, adjuvant), the term "able to augment the immunogenicity" refers to the ability to enhance the immunogenicity of an antigen or the ability to extend the duration of the immune response to an antigen, or both.

The phrase "enhance immune response" within the meaning of the present invention refers to the property or process of increasing the scale and/or efficiency of immunoreactivity to a given antigen, said immunoreactivity being either humoral or cellular immunity, or both. An immune response is believed to be enhanced, if any measurable parameter of antigen-specific immunoreactivity (*e.g.*, antibody titer, T cell production) is increased at least two-fold, preferably ten-fold, most preferably thirly-fold.

The term "therapeutically effective" applied to dose or amount refers to that quantity of a compound or pharmaceutical composition or vaccine that is sufficient to result in a desired activity upon administration to a mammal in need thereof. As used herein with respect to adjuvant-and antigen-containing compositions or vaccines, the term "therapeutically effective amount/dose" is used interchangeably with the term "immunogenically effective amount/dose" and refers to the amount/dose of a compound (*e.g.*, an antigen and/or an adjuvant comprising a γδ T cell activating compound) or pharmaceutical composition or vaccine that is sufficient to produce an effective immune response upon administration to a mammal.

The phrase "pharmaceutically acceptable", as used in connection with compositions of the invention, refers to molecular entities and other ingredients of such compositions that are physiologically tolerable and do not typically produce untoward reactions when administered to a human. Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical or vaccine compositions of the invention refers to a diluent, excipient, or vehicle with which a compound (*e.g.*, an antigen and/or an adjuvant comprising glycosylceramide) is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution, saline solutions, and aqueous dextrose and glycerol solutions are preferably employed as carriers, particularly for injectable solutions. Suitable pharmaceutical carriers are described in "Remington's Pharmaceutical Sciences" by E. W. Martin, $18^{th}$ Edition.

The term "native antibodies" or "immunoglobulins" refers to usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain (VL) at one end and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains (Clothia et al., J Mol. Biol., 186: 651-663, 1985; Novotny and Haber, Proc. Natl. Acad. Sci. USA,

82: 4592-4596, 1985).

The term "antibody" or "Ab" is used in the broadest sense and specifically covers not only native antibodies but also single monoclonal antibodies (including agonist and antagonist antibodies), antibody compositions with polyepitopic specificity, as well as antibody fragments (*e.g.*, Fab, F(ab')2, scFv and Fv), so long as they exhibit the desired biological activity.

"Cytokine" is a generic term for a group of proteins released by one cell population which act on another cell population as intercellular mediators. Examples of such cytokines are lymphokines, monokines, and traditional polypeptide hormones. Included among the cytokines are interferons (IFN, notably IFN-γ), interleukin (IL, notably IL-1, IL-2, IL-4, IL-10, IL-12), colony stimulating factors (CSF), thrombopoietin (TPO), erythropoietin (EPO), leukemia inhibitory factor (LIF), kit-ligand, growth hormones (GH), insulin-like growth factors (IGF), parathyroid hormone, thyroxine, insulin, relaxin, follicle stimulating hormone (FSH), thyroid stimulating hormone (TSH), leutinizing hormone (LH), hematopoietic growth factor, hepatic growth factor, fibroblast growth factors (FGF), prolactin, placental lactogen, tumor necrosis factors (TNF), mullerian-inhibiting substance, mouse gonadotropin-associated peptide, inhibin, activin, vascular endothelial growth factor (VEGF), integrin, nerve growth factors (NGF), platelet growth factor, transforming growth factors (TGF), osteoinductive factors, etc.

The term "subj ect" as used herein refers to an animal having an immune system, preferably a mammal or more preferably a primate. Preferably the term refers to humans.

The term "about" or "approximately" usually means within 20%, more preferably within 10%, and most preferably still within 5% of a given value or range. Alternatively, especially in biological systems (*e.g.*, when measuring an immune response), the term "about" means within about a log (*i.e.*, an order of magnitude) preferably within a factor of two of a given value.

The terms "vector", "cloning vector", and "expression vector" mean the vehicle by which a DNA or RNA sequence (*e.g.*, a foreign gene) can be introduced into a host cell, so as to transform the host and promote expression (*e.g.*, transcription and/or translation) of the introduced sequence. Vectors include plasmids, phages, viruses, etc.

In accordance with the present invention there may be employed conventional molecular biology, microbiology, and recombinant DNA techniques within the skill of the art. Such techniques are well-known and are explained fully in the literature. See, *e.g.*, Sambrook, Fritsch and Maniatis, Molecular Cloning: A Laboratory Manual, Second Edition (1989) Cold Spring Harbor Laboratory Press, Cold Spring Harbor, New York (herein "Sambrook *et al.*, 1989"); DNA Cloning: A Practical Approach, Volumes I and II (D. N. Glover ed. 1985); Oligonucleotide Synthesis (M. J. Gait ed. 1984); Nucleic Acid Hybridization [B. D. Hames & S. J. Higgins eds. (1985)]; Transcription And Translation [B. D. Hames & S. J. Higgins, eds. (1984)]; Animal Cell Culture [R. L Freshney, ed. (1986)]; Immobilized Cells And Enzymes [IRL Press, (1986)]; B. Perbal, A Practical Guide To Molecular Cloning (1984); F. M. Ausubel et al. (eds.), Current Protocols in Molecular Biology, John Wiley & Sons, Inc. (1994).

A "nucleic acid molecule" (or alternatively "nucleic acid") refers to the phosphate ester polymeric form of ribonucleosides (adenosine, guanosine, uridine, or cytidine: "RNA molecules") or deoxyribonucleosides (deoxyadenosine, deoxyguanosine, deoxythymidine, or deoxycytidine: "DNA molecules"), or any phosphoester analogs thereof, such as phosphorothioates and thioesters, in either single stranded form, or a double-stranded helix. Oligonucleotides (having fewer than 100 nucleotide constituent units) or polynucleotides are included within the defined term as well as double stranded DNA-DNA, DNA-RNA, and RNA-RNA helices. This term, for instance, includes double-stranded DNA found, inter alia, in linear (*e.g.*, restriction fragments) or circular DNA molecules, plasmids, and chromosomes. In discussing the structure of particular double-stranded DNA molecules, sequences may be described herein according to the normal convention of giving only the sequence in the 5' to 3' direction along the nontranscribed strand of DNA (*i.e.*, the strand having a sequence homologous to the mRNA). A "recombinant DNA molecule" is a DNA molecule that has undergone a molecular biological manipulation.

As used herein, the term "polypeptide" refers to an amino acid-based polymer, which can be encoded by a nucleic acid or prepared synthetically. Polypeptides can be proteins, protein fragments, chimeric proteins, etc. Generally, the term "protein" refers to a polypeptide expressed endogenously in a cell. Generally, a DNA sequence encoding a particular protein or enzyme is "transcribed" into a corresponding sequence of mRNA. The mRNA sequence is, in turn, "translated" into the sequence of amino acids which form a protein. An "amino acid sequence" is any chain of two or more amino acids. The term "peptide" is usually used for amino acid-based polymers having fewer than 100 amino acid constituent units, whereas the term "polypeptide" is reserved for polymers having at least 100 such units. Herein, however, "polypeptide" will be the generic term.

As used herein, an immunogenic composition is a composition that elicits an immune response in a mammal to which the composition is administered. The elicited immune response can be humoral or cell-mediated. As used herein, a vaccine composition is a composition which elicits an immune response in a mammal to whom the composition is administered and which protects the mammal from subsequent challenge or infection with the antigen of the composition or with a related organism. As used herein, "protection", for example protection against a virus, refers to generation of an immune response in the mammal (*e.g.*, primate) which is protective (partially or totally) against manifestations of the

disease caused by the antigen in the composition or a related organism, *e.g.* the virus. A vertebrate that is protected against disease caused by a virus may be infected with the virus, but to a lesser degree than would occur without immunization; may be infected with the virus, but does not exhibit disease symptoms; or may be infected with virus, but exhibits fewer disease symptoms than would occur without immunization. Alternatively, the vertebrate that is protected against disease caused by virus may not become infected with the virus at all, despite exposure to the virus.

**Adjuvant Composition, Vaccine Composition, and Uses Thereof**

The present invention provides a vaccine or adjuvant composition comprising at least a γδT cell activator of Formula I, preferably of Formula II or III, more preferably HDMAPP or CHDMAPP, and optionally a pharmaceutical acceptable carrier, diluent or excipient.

Examples of pharmaceutically acceptable carriers that can be used in accordance with the invention include typically large, slowly metabolizing macromolecules such as proteins, polysaccharides, polylactic acids, polyglycolic acids, polymeric amino acids, amino acid copolymers, and inactive virus particles. Examples of suitable carriers that also act as stabilizers for peptides include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, vehicles and auxiliary substances is available in REMINGTONS PHARMACEUTICAL SCIENCES (Mack Pub. Co., N. J. 1991). In a preferred embodiment, the pharmaceutical carrier is in the form of a lipid dispersion, more preferably the lipid dispersion consists of liposomes. Such carriers are well known to those of ordinary skill in the art. Pharmaceutically acceptable excipients include, but are not limited to, liquids such as water, saline, polyethyleneglycol, hyaluronic acid, glycerol and ethanol.

The present invention encompasses the use of a composition comprising at least a γδT cell activator of Formula I, preferably of Formula II or III, more preferably HDMAPP or CHDMAPP, as a vaccine adjuvant. The invention also concerns a vaccine adjuvant as such comprising a composition comprising at least one γδT cell activator of Formula I, preferably of Formula 11 or III, more preferably HDMAPP or CHDMAPP.

More particularly, the invention relates to a vaccine composition comprising a therapeutically effective amount of a vaccine and an amount of a γδT cell activator of Formula I, preferably of Formula II or III, more preferably HDMAPP or CHDMAPP sufficient to augment an immune response or preferably to enhance an immune response.

The adjuvant of the invention can be administered as part of a pharmaceutical or vaccine composition comprising an antigen or as a separate formulation, which is administered conjointly with a second composition containing an antigen. In any of these compositions γδ T cell activator of Formula 1, preferably of Formula II or III, more preferably HDMAPP or CHDMAPP, can be combined with other adjuvants and/or excipients/carriers. These other adjuvants include, but are not limited to, oil-emulsion and emulsifier-based adjuvants such as complete Freund's adjuvant, incomplete Freund's adjuvant, MF59, or SAF; mineral gels such as aluminum hydroxide (alum), aluminum phosphate or calcium phosphate; microbially-derived adjuvants such as cholera toxin (CT), pertussis toxin, *Escherichia coli* heat-labile toxin (LT), mutant toxins (*e.g.*, LTK63 or LTR72), Bacille Calmette-Guerin (BCG), *Corynebacterium parvum,* DNA CpG motifs, muramyl dipeptide, or monophosphoryl lipid A; particulate adjuvants such as immunostimulatory complexes (ISCOMs), liposomes, biodegradable microspheres, or saponins (*e.g.*, QS-21); synthetic adjuvants such as nonionic block copolymers, muramyl peptide analogues (*e.g.*, N-acetyl-muramyl-L-threonyl-D-isoglutamine [thr-MDP], N-acetyl-nor-muramyl-L-alanyl-D-isoglutamine, N-acetylmuramyl-L-alanyl-D-isoglutaminyl-L-alanine-2-[1'-2'-dipalmitoyl-sn-glycero-3-hydroxy-phospho-ryloxy]-ethylamine), polyphosphazenes, or synthetic polynucleotides, and surface active substances such as lysolecithin, pluronic polyols, polyanions, peptides, hydrocarbon emulsions, or keyhole limpet hemocyanins (KLH). Other preferred adjuvants include cytokines. Certain cytokines, for example TRANCE, flt-3L, and CD40L, enhance the immuno-stimulatory capacity of APCs. Non-limiting examples of cytokines, which may be used alone or in combination include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1 α), interleukin-11 (IL-11), MIP-1a, leukemia inhibitory factor (LIP), c-kit, ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme, Genentech, Amgen and Immunex. Preferably, these additional adjuvants are also pharmaceutically acceptable for use in humans.

An adjuvant compound of Formula I may be administered with a priming dose of antigen. An adjuvant compound of Formula I may then be administered again with the boost dose. Alternatively, an adjuvant compound of Formula I is administered with a boost dose of antigen. In another aspect, an adjuvant compound of Formula I is administered with a boost dose of antigen, but not with the priming dose of antigen. A "prime dose" is the first dose of antigen administered to the subject. In the case of a subject that has an infection the prime dose may be the initial exposure of the subject to the infectious microbe and thus the adjuvant is administered to the subject with the boost dose. A "boost dose" is a second or third, etc, dose of antigen administered to a subject that has already been exposed to the antigen. In some

cases the prime dose administered with the adjuvant is so effective that a boost dose is not required to protect a subject at risk of infection from being infected.

The vaccine composition is preferably a pharmaceutical composition comprising one or several antigens (or antigenic molecules), for combined, separate or sequential use. Antigens, as further described throughout the present disclosure, can be for example in the form of a synthetic or natural peptide, a lipid, a recombinant polypeptide, a nucleic acid, a killed, inactivated or attenuated pathogen, microorganism, or parasite. A vaccine can be, for example, an antigen such as protein or peptide in enriched or purified form, or an antigen delivered by a vector, such as for example, an adenoviral vector, Sindbis virus vector, or pox virus vector. An antigen can be entire protein or any epitope-containing fragment or portion thereof, particularly peptides that are presented to the immune system through MHC class I or MHC class II molecules. Examples include any viral antigen, bacterial antigen, parasite antigen, tumor antigen, etc.

There are at least six major types of vaccines: inactivated vaccines, attenuated vaccines, toxoids, antigenic extracts (purified or partially purified antigen), synthetic vaccines, and genetically engineered vaccines. Thus, a mammalian host may be vaccinated (immunized) against polio by the use of either an inactivated or attenuated vaccine, whereas vaccination (immunization) against tetanus is via the use of tetanus toxoid. Vaccination against a microbial infection may be by the use of an inactivated agent (*e.g.,* killed *Bordetella pertussis* in the case of pertussis vaccine), attenuated agent (*e.g.*, BCG in the case of tuberculosis vaccine), or purified antigen (*e.g.*, purified polysaccharide from *Neisseria meninaitidis* in the case of meningitis vaccine). The prevention of the growth or establishment of a tumor may be accomplished through the use of a purified or partially purified antigen vaccine, or by the use of the tumor cells themselves (in a protocol analogous to the use of attenuated or killed microbial vaccines).

A vaccine composition as described by the invention can be used to immunize humans or animals against different diseases (adjuvant). It is contemplated that the organism, host, or subject to which the compositions will be administered will be an animal or human.

A vaccine composition according to the invention may also be used in a subject for prophylactic or therapeutic stimulation of B or T lymphocyte development and proliferation, for enhancement of global and/or specific immuno-reconstitution, or for enhancement of humoral and/or cellular immune responses. For example, the vaccine composition can be used to prevent or reduce opportunistic infections in immuno-deficient patients.

The subject to whom the composition is administered may be naive or alternatively currently infected or diseased, or recovered from a past infection or disease state. The composition may be administered more than once, and in a specific embodiment at least twice or thrice. The administration of the vaccine composition can be a priming or a boosting administration.

The compositions can be administered to humans and animals either orally, rectally, parenterally (intravenous, by intramuscularly or subcutaneously), intracisternally, intravaginally, intraperitoneally, locally (powders, ointments or drops), or as a buccal or nasal spray.

The present invention is directed to a method of improving vaccine potency in a mammalian host comprising administering a therapeutically effective amount of a γδT cell activator of Formula I, preferably of Formula II or III, more preferably HDMAPP or CHDMAPP. Preferably, said γδT cell activator is as a pharmaceutical composition comprising said γδT cell activator and a pharmaceutically acceptable carrier.

More particularly, the present invention is directed to a method of improving the potency of a vaccine in a subject comprising the steps of:

administering to said subject a therapeutically effective amount of a vaccine; and,
administering to said subject an immune response enhancing amount of a γδ T cell activator of Formula I, preferably of Formula II or III, more preferably HDMAPP or CHDMAPP.

Preferably, the vaccine composition comprising one or several antigens and the γδ T cell activator of Formula I, preferably of Formula II or III, more preferably HDMAPP or CHDMAPP, are administered simultaneously. Alternatively, the vaccine composition and the γδ T cell activator of Formula I, preferably of Formula II or III, more preferably HDMAPP or CHDMAPP, are administered sequentially. Preferably the antigen-containing composition and the γδ T cell activator are administered with 48 hours of one another, preferably within 24 hours, 12 hours or 6 hours of one another.

The present invention also concerns a method of improving the potency of a vaccine comprising adding to said vaccine a potency improving amount of γδ T cell activator of Formula I, preferably of Formula II or III, more preferably HDMAPP or CHDMAPP.

The present invention relates to the use of a γδ T cell activator of Formula I, preferably of Formula II or III, more preferably of Formula IV or V, for the preparation of a medicament for the treatment and/or prevention of a disease whereby stimulation of the Thl and/or CTL response is needed. Preferably, said disease is selected from the group consisting of cancer, infections and autoimmune diseases. Indeed, the γδ T cell activators of Formula I, preferably of Formula II or III, more preferably of Formula IV or V, are used as an adjuvant to elicit dendritic cell maturation *in vivo.*

Another object of the invention relates to the use of a γδ T cell activator of Formula I, preferably of Formula II

or III, more preferably of Formula IV or V, for the manufacture of a pharmaceutical composition to enhance or augment an immune response.

A further particular object of the present invention concerns a method of causing or enhancing or augmenting an antigen-specific immune response in a subject, comprising administering to a subject said antigen and a composition comprising a γδ T cell activator of Formula I, preferably of Formula II or III, more preferably of Formula IV or V. The composition may be administered simultaneously, a few days before or sequentially with said antigen in order to obtain and/or stimulate an antigen-specific immune response in a subject.

*Microbial infections*

Preferably, said compositions of the invention prevent a microbial infection. Said microbial infection is caused by a microbe selected from the group consisting of viruses, fungi, yeast, bacteria, and protozoa.

A "microbial antigen" as used herein is an antigen of a microorganism and includes but is not limited to infectious virus, infectious bacteria, infectious parasites and infectious fungi. Such antigens include the intact microorganism as well as natural isolates and fragments or derivatives thereof and also synthetic compounds which are identical to or similar to natural microorganism antigens and induce an immune response specific for that microorganism. A compound is similar to a natural microorganism antigen if it induces an immune response (humoral and/or cellular) to a natural microorganism antigen. Most such antigens are used routinely in the art and are well known to those of ordinary skill in the art. Another example is a peptide mimic of a polysaccharide antigen.

Vaccines and antigens may be derived from infectious virus of both human and non-human vertebrates, include retroviruses, RNA viruses and DNA viruses. This group of retroviruses includes both simple retroviruses and complex retroviruses. The simple retroviruses include the subgroups of B-type retroviruses, C-type retroviruses and D-type retroviruses. An example of a B-type retrovirus is mouse mammary tumor virus (MMTV). The C-type retroviruses include subgroups C-type group A (including Rous sarcoma virus (RSV), avian leukemia virus (ALV), and avian myeloblastosis virus (AMV)) and C-type group B (including murine leukemia virus (MLV), feline leukemia virus (FeLV), murine sarcoma virus (MSV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, but also include HIV-2, SIV, Visna virus, feline immunodeficiency virus (FIV), and equine infectious anemia virus (EIAV). The T-cell leukemia viruses include HTLV-1, HTLV-II, simian T-cell leukemia virus (STLV), and bovine leukemia virus (BLV). The foamy viruses include human foamy virus (HFV), simian foamy virus (SFV) and bovine foamy virus (BFV).

Examples of other RNA viruses that are antigens in mammals include, but are not limited to, the following: members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, murine rotavirus, simian rotavirus, bovine or ovine rotavirus, avian rotavirus); the family Picornaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses , the genus Cardiovirus (Encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); the family Rhabdoviridae, including the genus Vesiculovirus (VSV), Chandipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Mouse Hepatitis virus, Human enteric corona virus, and Feline infectious peritonitis (Feline coronavirus).

Illustrative DNA viruses that are antigens in mammals include, but are not limited to: the family Poxviridae, including the genus Orthopoxvirus (Variola major, Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the alpha-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesvirises (Human cytomegalovirus and cytomegaloviruses of swine, monkeys and rodents); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc). Finally, DNA viruses may include viruses which do not fit into the above families such as Kuru and Creutzfeldt-Jacob disease viruses and chronic infectious neuropathic agents (CHINA virus).

Specific examples of HIV antigen can be, without any limitation, one or several antigens derived from a product selected from the group consisting of Tat, gp120, gp160, gag, pol, protease, and nef. Preferably, the HIV antigen is an antigen derived from gp 120.

Other preferred exemplary antigens are HPV antigens from any strain of HPV. HPV expresses six or seven non-structural and two structural proteins. Viral capsid proteins L1 and L2 are the late structural proteins. L1 is the major capsid protein, the amino acid sequence of which is highly conserved among different HPV types. There are seven early non-structural proteins. Proteins E1, E2, and E4 play an important role in virus replication. Protein E4 also plays a role in virus maturation. The role of E5 is less well known. Proteins E6 and E7 are oncoproteins critical for viral replication, as well as for host cell immortalization and transformation. Fusion proteins of the invention can contain either the entire sequence of an HPV protein or a fragment thereof, *e.g.*, a fragment of at least 8 amino acids. In one embodiment, the HPV antigenic sequence is derived from a "high risk" HPV, such as HPV 16 or HPV 18 E7 protein. The HPV antigenic sequence can include an MHC-binding epitope, *e.g.*, an MHC class I and/or an MHC class II binding epitope.

Further preferred exemplary antigens are those obtained or derived from the hepatitis family of viruses, including hepatitis A virus (HAV), hepatitis B virus (BBV), hepatitis C virus (HCV), the delta hepatitis virus (HDV), hepatitis E virus (BEV) and hepatitis G virus (HGV). See, e. g., International Publication Nos. WO 89/04669; WO 90/11089; and WO 90/14436. The HCV genome encodes several viral proteins, including E I and E2. See, *e.g.*, Houghton et al. (1991) Hepatology 14: 381-388. Nucleic acid molecules containing sequences encoding these proteins, as well as antigenic fragments thereof, will find use in the present methods. Similarly, the coding sequence for the 8-antigen from HDV is

known (see U. S. Patent No. 5,378,814). In like manner, a wide variety of proteins from the herpesvirus family can be used as antigens in the present invention, including proteins derived from herpes simplex virus (HSV) types 1 and 2, such as HSV- I and HSV-2 glycoproteins gB, gD and gH; antigens from varicella zoster virus (VZV), Epstein-Barr virus (EBV) and cytomegalovirus (CMV) including CMV gB, and gH; and antigens fi-om other human herpesviruses such as HHV6 and HAV7. (See, e. g. Chee et al. (1990) Cytomegaloviruses (J. K. McDougall, ed., Springer Verlag, pp. 125-169; McGeoch et al. (1988) J. Gen. Virol. 69: 1531-1574; U. S. Patent No. 5,171,568; Baer et al. (1984) Nature 310: 207-21 1; and Davison et al. (1986) J. Gen. Virol. 67: 1759 )

Antigens or vaccines may be derived from respiratory syncytial virus (RSV), a negative strand virus of the paramyxoviridae family and a major cause of lower pulmonary tract disease, particularly in young children and infants. RSV contains two prominent outer envelope glycoproteins, (fusion (F) protein and attachment (G) protein), that are important for viral infectivity and thus serve as reasonable targets for the design of a subunit vaccine to RSV. The wild type (native) nucleotide and amino acid sequences of the RSV F protein are known in the art (Collins et al., Proc. Natl. Acad. Sci (USA) 81:7683-7687 (1984); U.S. Patent No. 5,639,853; U.S. Patent No. 5,723,130). Preferred RSV proteins suitable for use in the invention include the complete RSV F protein as well as functional portions of the RSV F protein. For example, a functional portion can be a portion of the protein which retains the ability to induce an antibody response when administered to a mammal. Examples of such immunogenic portions are polypeptides comprising amino acid positions 283-315, 289-315 and 294-299 of the RSV F protein. These regions include an epitope of the RSV F protein which elicits both neutralizing and antifusion antibodies (U.S. Patent 5,639,853). Alternatively, an RSV F protein in its native dimeric form (140 kD) may be used (U.S. Patent 5,223,254).

Other exemplary vaccines used in accordance with the adjuvants of the invention include Influenza Virus Vaccines. Trivalent A & B Live, Cold Adapted or FluMist (Medimmune Vaccines) is an aqueous nasal spray trivalent formulation of natural recombinant (re-assortment; not gene spliced) cold-adapted temperature-sensitive attenuated nonpathogenic live influenza viruses having immunogenic viral coat proteins (hemagglutinin and neuraminidase) from representative virulent epidemic wild-type influenza strains and an influenza virus core with six attenuating gene mutations. Preferred recombinant cold-adapted/temperature-sensitive influenza virus strains that can be used as vaccines have a viral coat presenting influenza virus hemagglutinin (HA) and neuraminidase (NA) immunogenic epitopes from a virulent influenza strain along with an attenuated influenza virus core. The HA and NA RNA sequences of an attenuated master donor virus (MDV) are replaced with HA and NA RNA sequences from epidemic wild-type influenza strains. Temperature sensitivity is conferred to the MDV by modification of the polymerase Basic Protein 2 (PB2) gene, which encodes a 759 amino acid polypeptide that is one of the three proteins comprising the RNA-dependent polymerase complex of influenza virus. Viral RNA replication is dependent on PB2 (along with PB1, PA, and NP). The three polymerase proteins, PB1, PB2, and PA, form a trimolecular complex in the nuclei of infected cells

Other vaccines and antigens may be derived from bacteria, parasites or yeast. Examples of suitable species include *Neisseria* spp, including *N. gonorrhea* and *N. meningitidis* (for example, capsular polysaccharides and conjugates thereof, transferrin-binding proteins, lactoferrin binding proteins, PilC and adhesions can be used as antigens); *S. pyogenes* (for example M proteins or fragments thereof, C5A protease, lipoteichoic acids), *S. agalactiae*, *S. mutans*; *H. ducreyi*; *Moraxella* spp, including *M. catarrhalis*, also known as *Branhamella catarrhalis* (for example high and low molecular weight adhesins and invasins); *Bordetella* spp, including *B. pertussis* (for example pertactin, pertussis toxin or derivatives thereof, filamenteous hemagglutinin, adenylate cyclase, fimbriae), *B. parapertussis* and *B. bronchiseptica*; *Mycobacterium* spp., including *M. tuberculosis* (for example ESAT6, Antigen 85A, -B or -Q, *M. bovis*, *M. leprae*, *M. avium*, *M. paratuberculosis*, *M. smegmatis*; *Legionella* spp, including *L. pneumophila*; *Escherichia* spp, including enterotoxic *E. coli* (for example colonization factors, heat-labile toxin or derivatives thereof, heat-stable toxin or derivatives thereof), enterohemorragic *E. coli*, enteropathogenic *E. coli* (for example *Vibrio shiga* toxin-like toxin or derivatives thereof); *Vibrio* spp, including *V. cholera* (for example cholera toxin or derivatives thereof; *Shigella* spp, including *S. sonnei*, *S. dysenteriae*, *S. flexnerii*; *Yersinia* spp, including *Y. enterocolitica* (for example a Yop protein) , *Y. pestis*, *Y. pseudotuberculosis*; *Campylobacter* spp, including *C. jejuni* (for example toxins, adhesins and invasins) and *C coli*; *Salmonella* spp, including *S. typhip*, *S. paratyphi*, *S. choleraesuis*, *S. enteritidis*; *Listeria* spp., including *L. monocytogenes*; *Helicobacter* spp, including *H. pylori* (for example urease, catalase, vacuolating toxin); *Pseudomonas* spp, including *P. aeruginosa*; *Staphylococcus* spp., including *S. aureus*, *S. epidermidis*; *Enterococcus* spp., including *E. jaecalis*, *E. jaecium*; *Clostridium* spp., including *C*, *tetani* (for example tetanus toxin and derivatives thereof), *C. botulinum* (for example botulinum toxin and derivatives thereof, *C. difficile* (for example clostridium toxins A or B and derivatives thereof); *Bacillus* spp., including *B. anthracis* (for example botulinum toxin and derivatives thereof); *Corynebacterium* spp., including *C. diphtheriae* (for example diphtheria toxin and derivatives thereof); *Borrelia* spp., including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii*; *Ehrlichia* spp., including *E. equi* and the agent of the Human Granulocytic Ehrlichiosis; *Rickettsia* spp, including *R. rickettsii*; *Chlamydia* spp., including *C. trachomatis* (for example MOMP, heparin-binding proteins), *C. pneumoniae* (for example MONT, heparin-binding proteins), *C. psittaci*; *Leptospira* spp., including *L. interrogans*; *Treponema* spp., including *T. pallidum* (for

example the rare outer membrane proteins), *T. denticola*, *T. hyodysenteriae*; or species derived from parasites such as *Plasmodium* spp., including *P. falciparum*; *Toxoplasma* spp., including *T. gondii* (for example SAG2, SAG3, Yg34); *Entamoeba* spp., including *E. histolytica*; *Babesia* spp., including *B. microti*; *Trypanosoma* spp., including *T. cruzi*; *Giardia* spp., including *G. lamblia*; *Leshmania* spp., including *L. major*; *Pneumocystis* spp., including *P. carinii*; *Trichomonas* spp., including *T. vaginalis*; *Schisostoma* spp., including *S. mansoni*, or species derived from yeast such as *Candida* spp., including *C albicans*; *Cryptococcus* spp., including *C neoformans*.

Examples of preferred specific antigens for M. tuberculosis are for example Th Ra, Th H9, Th Ra35, Th38-1, Erd 14, DPV, MTI, MSL, mTTC2 and hTCC1 (WO 99/51748). Proteins for M tuberculosis also include fusion proteins and variants thereof where at least two, preferably three polypeptides of M. tuberculosis are fused into a larger protein.

Examples of preferred antigens for Chlamydia include for example the High Molecular Weight Protein (HWMP) (WO 99/17741), ORF3 (EP 366 412), and putative membrane proteins (Pmps). Other *Chlamydia* antigens of the vaccine formulation can be selected from the group described in WO 99/28475.

Other preferred bacterial vaccines comprise antigens derived from *Streptococcus* spp, including *S. pneumoniae* (for example capsular polysaccharides and conjugates thereof, Psa.A, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951).

*Cancer vaccines*

A "cancer antigen" or "tumor antigen" as used herein is a compound, such as a peptide, associated with a tumor or cancer cell surface and which is capable of provoking an immune response when expressed on the surface of an antigen presenting cell in the context of an MHC molecule. Cancer antigens can be prepared from cancer cells either by preparing crude extracts of cancer cells, for example, as described in Cohen, et al., 1994, Cancer Research, 54: 1055, by partially purifying the antigens, by recombinant technology, or by de novo synthesis of known antigens. Cancer antigens include antigens that are recombinantly an immunogenic portion of or a whole tumor or cancer. Such antigens can be isolated or prepared recombinantly or by any other means known in the art.

The formulations may also contain a tumour antigen and be useful for the immunotherapeutic treatment of cancers. For example, the adjuvant formulation finds utility with tumour rejection antigens such as those for prostate, breast, colorectal, lung, pancreatic, renal or melanoma cancers. Exemplary antigens include MAGE 1 and MAGE 3 or other MAGE antigens (for the treatment of melanoma), PRAME, BAGE, or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumor types, such as melanoma, lung carcinoma, sarcoma and bladder carcinoma. Other tumour-specific antigens are suitable for use with the adjuvants of the present invention and include, but are not restricted to tumour-specific gangliosides, Prostate specific antigen (PSA) or Her-2/neu, KSA (GA733), PAP, mammaglobin, MUC-1, carcinoembryonic antigen (CEA). Accordingly in one aspect of the present invention there is provided a vaccine comprising an adjuvant composition according to the invention and a tumour rejection antigen.

It is a particularly preferred aspect of the present invention that the vaccines comprise a tumor antigen; such vaccines are surprisingly potent in the therapy of cancer such as prostrate, breast, colorectal, lung, pancreatic, renal, ovarian or melanoma cancers.

Accordingly, the formulations may contain tumour-associated antigen, as well as antigens associated with tumor-support mechanisms (*e.g.* angiogenesis, tumor invasion). Additionally, antigens particularly relevant for vaccines in the therapy of cancer also comprise Prostate-specific membrane antigen (PSMA), Prostate Stem Cell Antigen (PSCA), tyrosinase, survivin, NY-ES01, prostase, PS108 (WO 98/50567), RAGE, LAGE, HAGE. Additionally said antigen may be a self peptide hormone such as whole length Gonadotropin hormone releasing hormone (GnRH, WO 95/20600), a short 10 amino acid long peptide, useful in the treatment of many cancers, or in immuno-castration.

It is foreseen that compositions of the present invention will be used to formulate vaccines containing antigens derived from Borrelia sp. Vaccines of the present invention may be used for the prophylaxis or therapy of allergy. Such vaccines would comprise allergen specific (for example Der p 1) and allergen non-specific antigens (for example peptides derived from human IgE, including but not restricted to the stanworth decapeptide (EP 0 477 231 B1)).

Vaccines of the present invention may also be used for the prophylaxis or therapy of chronic disorders others than allergy, cancer or infectious diseases. Such chronic disorders are diseases such as atherosclerosis, and Alzheimer. Antigens relevant for the prophylaxis and the therapy of patients susceptible to or suffering from Alzheimer neurodegenerative disease are, in particular, the N terminal 39 -43 amino acid fragment (AP) of the amyloid precursor protein and smaller fragments (WO 99/27944).

Each of the foregoing lists is illustrative, and is not intended to be limiting.

**High-potency γδT Cell Activators**

The terms "γδT lymphocyte activating compound", "γδT cell activating compound", "γδT lymphocyte activator" and "γδT cell activator", used interchangeably, designate a molecule that can activate γδT lymphocytes. More particularly, the term γδT lymphocyte activating compound designates a molecule produced ex vivo or *in vitro*. It is more preferably a ligand of the T receptor of γδT lymphocytes. The activator may be of various natures, such as a peptide, lipid, small molecule, etc. It may be a purified or otherwise artificially produced (*e.g.*, by chemical synthesis, or by microbiological process) endogenous ligand, or a fragment or derivative thereof, or an antibody having substantially the same antigenic specificity. The activator is most preferably a synthetic chemical compound capable of selectively activating Vγ9Vδ2 T lymphocytes. Selective activation of Vγ9Vδ2 T lymphocytes indicates that the compound has a selective action towards specific cell populations, and essentially does not activate other T cell sub-types, such as Vδ1 T cells. Such selectivity, as disclosed in the present application, suggests that preferred compounds can cause a selective or targeted activation of, proliferation of or other biological activity of Vγ9Vδ2 T lymphocytes.

Preferably a γδ T lymphocyte activator is a compound capable of regulating the activity of a γδ T cell in a population of γδ T cell clones in culture. The γδ T lymphocyte activator is capable of regulating the activity of a γδ T cell population of γδ T cell clones in a millimolar concentration, preferably when the γδ T cell activator is present in culture at a concentration of less than 100 mM. Optionally a γδ T lymphocyte is capable of regulating the activity of a γδ T cell in a population of γδ T cell clones at millimolar concentration, preferably when the γδ T cell activator is present in culture at a concentration of less than 10 mM, or more preferably less than 1 mM. Regulating the activity of a γδ T cell can be assessed by any suitable means, preferably by assessing cytokine secretion, most preferably TNF-α secretion as described herein. Methods for obtaining a population of pure γδ T cell clones is described in Davodeau et al., ((1993) J. Immunology 151(3): 1214-1223) and Moreau et al., ((1986) J. Clin. Invest. 78:874).

Preferably the compound is capable of causing at least a 20%, 50% or greater increase in the number of γδ T cells in culture, or more preferably at least a 2-fold increase in the number of γδ T cells in culture.

In a preferred aspect, the γδ T cell activator may increase the biological activity of γδ T cells, preferably increasing the activation of γδ T cells, particularly increasing cytokine secretion from γδ T cells, with or without also stimulating the expansion of γδ T cells.

Cytokine secretion can be assessed using any appropriate *in vitro* assay, or those provided in the examples herein. For example, cytokine secretion can be determined according to the methods described in Espinosa et al. (J. Biol. Chem., 2001, Vol. 276, Issue 21, 18337-18344), describing measurement of TNF-α release in a bioassay using TNF-α-sensitive cells. Briefly, $10^4$ γδT cells/well are incubated with stimulus plus 25 units of IL2/well in 100 μl of culture medium during 24 h at 37 °C. Then, 50 μl of supernatant are added to 50 μl of WEHI cells plated at $3 \times 10^4$ cells/well in culture medium plus actinomycin D (2 μg/ml) and LiCl (40 mM) and incubated for 20 h at 37 °C. Viability of the TNF-α-sensitive cells is measured with a 3-(4,5-dimethylthiazol-2-yl)-2,5-diphenyltetrazolium bromide assay. Fifty microliters of 3-(4,5-dimethylthiazol-2-yl)-2,5-diphenyltetrazolium bromide (Sigma; 2.5 mg/ml in phosphate-buffered saline) per well are added, and after 4 h of incubation at 37 °C, 50 μl of solubilization buffer (20% SDS, 66% dimethyl formamide, pH 4.7) are added, and absorbance (570 nm) is measured. Levels of TNF-α release are then calculated from a standard curve obtained using purified human rTNF-α (PeproTech, Inc., Rocky Hill, NJ). TNF-α detection can also be carried out according to the manufacturer's instructions (kit ref 1121, Immunotech-Beckman Coulter). Interferon-γ released by activated T cells is measured by a sandwich enzyme-linked immunosorbent assay. Gamma delta T cells ($5 \times 10^4$ γδT cells/well) are incubated with stimulus plus 25 units of IL2/well in 100 μl of culture medium during 24 h at 37 °C. Then, 50 μl of supernatant is harvested for enzyme-linked immunosorbent assay using mouse monoclonal antibodies (Biosource International, California).

The inventors disclose that γδ T cell activators having high *in vitro* and *in vivo* potency can be used according to the methods of the invention where the absence toxicity is requirement. A preferred compound is a γδ T cell activator that is: (a) capable of regulating the activity of a γδ. T cell in a population of γδ T cell clones at millimolar concentration, preferably when the γδ T cell activator is present in culture at a concentration of 10 mM or less; and (b) administered to a mammal, or in dosage form for administration to a mammal, at *in vitro* dosage of less than about 1 mg/kg, preferably between about 10 μg/kg and 100 μg/kg, or more preferably less than about 100 μg/kg or less than 10 μg/kg. Preferably regulating the activity of a γδ T cell refers to stimulating of cytokine production or cytokine release as described herein.

The γδT cell activators according to the present invention preferably comprise the compounds of formula (I):

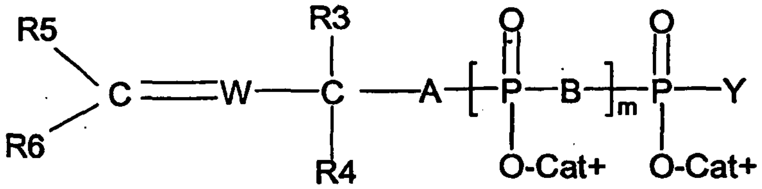

(I)

in which $R_3$, $R_4$, and $R_5$ , identical or different, are a hydrogen or $(C_1-C_3)$alkyl group or any other isosteric group thereof such as $CF_3$, W is -CH- or -N-, $R_6$ is an $(C_2-C_3)$acyl, an aldehyde, an $(C_1-C_3)$alcohol, or an $(C_2-C_3)$ester, Cat+ represents one (or several, identical or different) organic or mineral cation(s) (including the proton), B is O or NH, m is an integer from 1 to 3, A is O, NH, CHF, $CF_2$ or $CH_2$ or any other isosteric group, and Y is $O^-$Cat+, a nucleoside, or a radical -A-R, wherein R is a linear, branched, or cyclic, aromatic or not, saturated or unsaturated, $C_1-C_{50}$ hydrocarbon group, optionally interrupted by at least one heteroatom, wherein said hydrocarbon group comprises an alkyl, an alkylenyl, or an alkynyl, preferably an alkyl or an alkylene, which can be substituted by one or several substituents selected from the group consisting of : an alkyl, an alkylenyl, an alkynyl, an epoxyalkyl, an aryl, an heterocycle, an alkoxy, an acyl, an alcohol, a carboxylic group (-COOH), an ester, an amine, an amino group ($-NH_2$), an amide ($-CONH_2$), an imine, a nitrile, an hydroxyl (-OH), a aldehyde group (-CHO), an halogen, an halogenoalkyl, a thiol (-SH), a thioalkyl, a sulfone, a sulfoxide, and a combination thereof.

In a particular embodiment, the substituents as defined above are substituted by at least one of the substituents as specified above.

Preferably, the substituents are selected from the group consisting of : an $(C_1-C_6)$alkyl, an $(C_2-C_6)$alkylenyl, an $(C_2-C_6)$alkynyl, an $(C_2-C_6)$epoxyalkyl, an aryl, an heterocycle, an $(C_1-C_6)$alkoxy, an $(C_2-C_6)$acyl, an $(C_1-C_6)$alcohol, a carboxylic group (-COOH), an $(C_2-C_6)$ester, an $(C_1-C_6)$amine, an amino group ($-NH_2$), an amide ($-CONH_2$), an $(C_1-C_6)$ imine, a nitrile, an hydroxyl (-OH), a aldehyde group (-CHO), an halogen, an $(C_1-C_6)$halogenoalkyl, a thiol (-SH), a $(C_1-C_6)$thioalkyl, a $(C_1-C_6)$sulfone, a $(C_1-C_6)$sulfoxide, and a combination thereof.

More preferably, the substituents are selected from the group consisting of : an $(C_1-C_6)$alkyl, an $(C_2-C_6)$epoxyalkyl, an $(C_2-C_6)$alkylenyl, an $(C_1-C_6)$alkoxy, an $(C_2-C_6)$acyl, an $(C_1-C_6)$alcohol, an $(C_2-C_6)$ester, an $(C_1-C_6)$amine, an $(C_1-C_6)$imine, an hydroxyl, a aldehyde group, an halogen, an $(C_1-C_6)$halogenoalkyl, and a combination thereof.

Still more preferably, the substituents are selected from the group consisting of : an $(C_3-C_6)$epoxyalkyl, an $(C_1-C_3)$alkoxy, an $(C_2-C_3)$acyl, an $(C_1-C_3)$alcohol, an $(C_2-C_3)$ester, an $(C_1-C_3)$amine, an $(C_1-C_3)$imine, an hydroxyl, an halogen, an $(C_1-C_3)$halogenoalkyl, and a combination thereof. Preferably, R is a $(C_3-C_{25})$hydrocarbon group. More preferably a $(C_5-C_{10})$hydrocarbon group.

In the context of the present invention, the term "alkyl" more specifically means a group such as methyl, ethyl, propyl, isopropyl, butyl, isobutyl, *tert*-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl and the other isomeric forms thereof. More specifically, $(C_1-C_6)$alkyl means methyl, ethyl, propyl, isopropyl, butyl, isobutyl, *tert*-butyl, pentyl, hexyl and the other isomeric forms thereof. More specifically, $(C_1-C_3)$alkyl means methyl, ethyl, propyl, or isopropyl.

The term "alkenyl" refers to an alkyl group defined hereinabove having at least one unsaturated ethylene bond and the term "alkynyl" refers to an alkyl group defined hereinabove having at least one unsaturated acetylene bond. $(C_2-C_6)$alkylene includes a ethenyl, a propenyl (1-propenyl or 2-propenyl), a 1- or 2- methylpropenyl, a butenyl (1-butenyl, 2-butenyl, or 3-butenyl), a methylbutenyl, a 2-ethylpropenyl, a pentenyl (1-pentenyl, 2-pentenyl, 3-pentenyl, 4-pentenyl), an hexenyl (1-hexenyl, 2-hexenyl, 3-hexenyl, 4-hexenyl, 5-hexenyl), and the other isomeric forms thereof. $(C_2-C_6)$alkynyl includes ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl and the other isomeric forms thereof.

The term "epoxyalkyl" refers to an alkyl group defined hereinabove having an epoxide group. More particularly, $(C_2-C_6)$epoxyalkyl includes epoxyethyl, epoxypropyl, epoxybutyl, epoxypentyl, epoxyhexyl and the other isomeric forms thereof. $(C_2-C_3)$epoxyalkyl includes epoxyethyl and epoxypropyl.

The "aryl" groups are mono-, bi- or tri-cyclic aromatic hydrocarbons having from 6 to 18 carbon atoms. Examples include a phenyl, α-naphthyl, β-naphthyl or anthracenyl group, in particular.

"Heterocycle" groups are groups containing 5 to 18 rings comprising one or more heteroatoms, preferably 1 to 5 endocyclic heteroatoms. They may be mono-, bi- or tri-cyclic. They may be aromatic or not. Preferably, and more specifically for $R_5$, they are aromatic heterocycles. Examples of aromatic heterocycles include pyridine, pyridazine, pyrimidine, pyrazine, furan, thiophene, pyrrole, oxazole, thiazole, isothiazole, imidazole, pyrazole, oxadiazole, triazole, thiadiazole and triazine groups. Examples of bicycles include in particular quinoline, isoquinoline and quinazoline groups (for two 6-membered rings) and indole, benzimidazole, benzoxazole, benzothiazole and indazole (for a 6-membered ring and a 5-membered ring). Nonaromatic heterocycles comprise in particular, piperazine, piperidine, etc.

"Alkoxy" groups correspond to the alkyl groups defined hereinabove bonded to the molecule by an - O- (ether) bond. $(C_1-C_6)$alkoxy includes methoxy, ethoxy, propyloxy, butyloxy, pentyloxy, hexyloxy and the other isomeric forms thereof. $(C_1-C_3)$alkoxy includes methoxy, ethoxy, propyloxy, and isopropyloxy.

"Alcyl" groups correspond to the alkyl groups defined hereinabove bonded to the molecule by an - CO- (carbonyl) group. $(C_2-C_6)$acyl includes acetyl, propylacyl, butylacyl, pentylacyl, hexylacyl and the other isomeric forms thereof. $(C_2-C_3)$acyl includes acetyl, propylacyl and isopropylacyl.

"Alcohol" groups correspond to the alkyl groups defined hereinabove containing at least one hydroxyl group. Alcohol can be primary, secondary or tertiary. $(C_1-C_6)$alcohol includes methanol, ethanol, propanol, butanol, pentanol, hexanol and the other isomeric forms thereof. $(C_1-C_3)$alcohol includes methanol, ethanol, propanol and isopropanol.

"Ester" groups correspond to the alkyl groups defined hereinabove bonded to the molecule by a - COO- (ester) bond. $(C_2-C_6)$ester includes methylester, ethylester, propylester, butylester, pentylester and the other isomeric forms thereof. $(C_2-C_3)$ester includes methylester and ethylester.

"Amine" groups correspond to the alkyl groups defined hereinabove bonded to the molecule by an - N- (amine) bond. $(C_1-C_6)$amine includes methylamine, ethylamine, propylamine, butylamine, pentylamine, hexylamine and the other isomeric forms thereof. $(C_1-C_3)$amine includes methylamine, ethylamine, and propylamine.

"Imine" groups correspond to the alkyl groups defined hereinabove having a (-C=N-) bond. $(C_1-C_6)$imine includes methylimine, ethylimine, propylimine, butylimine, pentylimine, hexylimine and the other isomeric forms thereof. $(C_1-C_3)$ imine includes methylimine, ethylimine, and propylimine.

The halogen can be Cl, Br, I, or F.

"Halogenoalkyl" groups correspond to the alkyl groups defined hereinabove having at least one halogen. The groups can be monohalogenated or polyhalogenated containing the same or different halogen atoms. For example, the group can be a trifluoroalkyl ($CF_3$-R). $(C_1-C_6)$halogenoalkyl includes halogenomethyl, halogenoethyl, halogenopropyl, halogenobutyl, halogenopentyl, halogenohexyl and the other isomeric forms thereof. $(C_1-C_3)$halogenoalkyl includes halogenomethyl, halogenoethyl, and halogenopropyl.

"Thioalkyl" groups correspond to the alkyl groups defined hereinabove bonded to the molecule by an -S- (thioether) bond. $(C_1-C_6)$thioalkyl includes thiomethyl, thioethyl, thiopropyl, thiobutyl, thiopentyl, thiohexyl and the other isomeric forms thereof. $(C_1-C_3)$thioalkyl includes thiomethyl, thioethyl, and thiopropyl.

"Sulfone" groups correspond to the alkyl groups defined hereinabove bonded to the molecule by an - SOO- (sulfone) bond. $(C_1-C_6)$sulfone includes methylsulfone, ethylsulfone, propylsulfone, butylsulfone, pentylsulfone, hexylsulfone and the other isomeric forms thereof. $(C_1-C_3)$sulfone includes methylsulfone, ethylsulfone and propylsulfone.

"Sulfoxide" groups correspond to the alkyl groups defined hereinabove bonded to the molecule by an -SO- (sulfoxide) group. $(C_1-C_6)$sulfoxide includes methylsulfoxide, ethylsulfoxide, propylsulfoxide, butylsulfoxide, pentylsulfoxide, hexylsulfoxide and the other isomeric forms thereof. $(C_1-C_3)$sulfoxide includes methylsulfoxide, ethylsulfoxide, propylsulfoxide and isopropylsulfoxide.

"Heteroatom" denotes N, S, or O.

"Nucleoside" includes adenosine, thymine, uridine, cytidine and guanosine.

An "isosteric group" refers to to elements, functional groups, substitutents, molecules or ions having different molecular formulae but exhibiting similar or identical physical properties. For example, $CF_3$ is an isosteric group of $CH_3$. Typically, two isosteric groups have similar or identical volumes and shapes.

Preferably, Y is $O^-Cat+$, or a nucleoside. More preferably, Y is $O^-Cat+$. Preferably, A is O or $CH_2$. More preferably, A is O. More preferably, $R_3$ and $R_5$ are a methyl or an isosteric group thereof, such as $CH_2F$, $CF_2H$ or $CF_3$ and $R_4$ is a hydrogen. Still more preferably, $R_3$ and $R_5$ are a methyl and $R_4$ is a hydrogen.More preferably, $R_6$ is -$CH_2$-OH, -CHO, -CO-$CH_3$ or -CO-O$CH_3$. Preferably, B is O. Preferably, m is 1 or 2. More preferably, m is 1. Optionally, the double-bond between W and C is in conformation trans (E) or cis (Z). More preferably, the double-bond between W and C is in conformation trans (E).

The group Y can allow design of a prodrug. Therefore, Y is enzymolabile group which can be cleaved in particular regions of the subject. The group Y can also be targeting group. In a preferred embodiment, Y is $O^-Cat+$, a group -A-R, or a radical selected from the group consisting of a nucleoside, a monosaccharide, an epoxide and a halohydrin. Preferably, Y is an enzymolabile group. Preferably, Y is $O^-Cat+$, a group -A-R, or a nucleoside. In a first preferred embodiment, Y is $O^-Cat+$. In a second preferred embodiment, Y is a nucleoside.

In a preferred embodiment, $Cat^+$ is $H^+$, $Na^+$, $NH_4^+$, $K^+$, $Li^+$, $(CH_3CH_2)_3NH^+$.

In a preferred embodiment, γδT cell activators comprise the compounds of formula (II) or (III) :

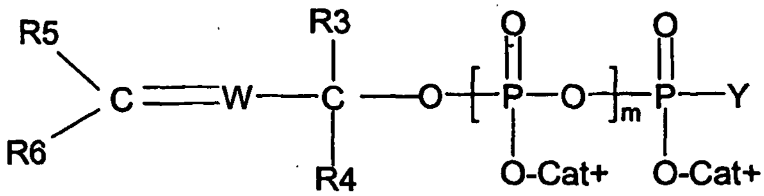

(II)

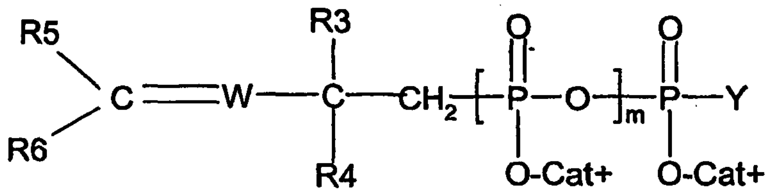

(III)

wherein $R_3$, $R_4$, and $R_5$, identical or different, are a hydrogen or ($C_1$-$C_3$)alkyl group or an isosteric group thereof, W is -CH- or -N-, $R_6$ is an ($C_2$-$C_3$)acyl, an aldehyde, an ($C_1$-$C_3$)alcohol, or an ($C_2$-$C_3$)ester, Cat+ represents one (or several, identical or different) organic or mineral cation(s) (including the proton), m is an integer from 1 to 3, and Y is $O^-$Cat+ or a nucleoside. Preferably, W is -CH-. Preferably, R3 and R4 are hydrogen. Preferably, R5 is a methyl or an isosteric group thereof such as $CF_3$. More preferably, R5 is a methyl. Preferably, R6 is -$CH_2$-OH.

In a preferred embodiment, γδT cell activator is the compound of formula (IV):

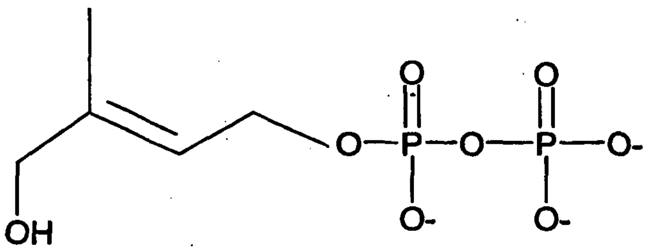

(IV) HDMAPP

(E)-4-hydroxy-3-methyl-2-butenyl pyrophosphate

In another preferred embodiment, the γδT cell activator is the compound of formula (V):

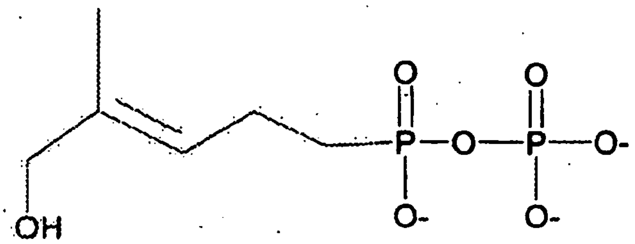

(V) CHDMAPP

(E)-5-hydroxy-4-methylpent-3-enyl pyrophosphonate

These compounds may be produced according to various techniques known per se in the art, some of which being disclosed in PCT Publication WO 03/009855 and corresponding US Publication US 2006-0030546.

In another preferred embodiment, the γδT cell activator is the compound of formula (VI), also referred to as

NHDMAPP described in -PCT Publication No. WO 05/054258 and U.S. Provisional patent application no. 60/579,237 filed June 15th, 2004.

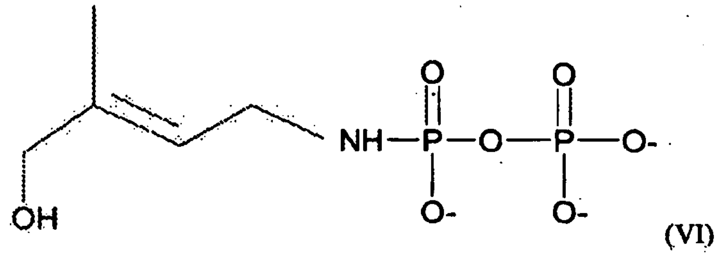

(VI)

Compounds comprising a nucleoside as Y group can be prepared, for example, by the following reactions. Depending on the type and reactivity of the functional groups provided by Y, the professional is able to adapt the following examples, if necessary including the phases of protection/non-protection of the sensitive functional groups or those that can interact with the coupling reaction.

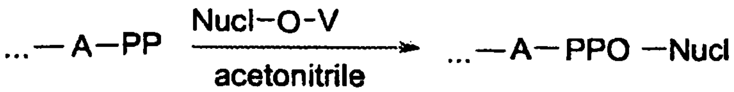


Reaction A

or

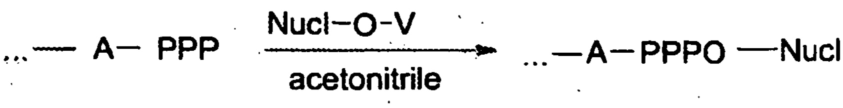


Reaction B

where -O-V is a good group beginning with V chosen, for example, from among tosyle, mesyle, triflyle, brosyle or bromium, PP represents the pyrophosphate group, PPP represents the triphosphate group, ...-A- has the above mentioned meaning wherein ... refers to

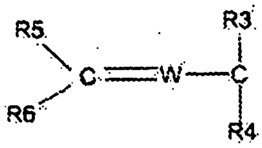

and Nucl is a nucleoside. Preferably, Nucl-O-V is selected from the group consisting of : 5'-O-Tosyladenosine, 5'-O-Tosyluridine, 5'-O-Tosylcytidine, 5'-O-Tosylthymidine or 5'-O-Tosyl-2'-deoxyadenosine.

Alternatively, compounds comprising a nucleoside as Y group can be prepared by the following reaction:

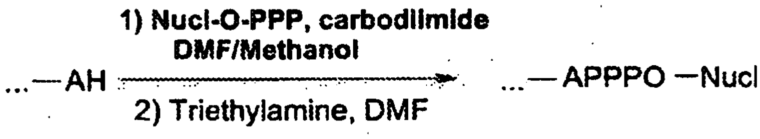


Reaction C

where PPP represents the triphosphate group, ...-A has the above mentioned meaning, DMF is dimethylformamide, and Nucl is a nucleoside. This reaction can be carried out in conditions similar to those described by Knorre *et al.*(1976), or by Bloom *et al.*, United States Patent No. 5,639,653 (1997), from alcohol and a nucleotide with formula Nucl-O-PPP.

*HDMAPP*

Since the isolation of HDMAPP from *E. coli* cells deficient in the lytB component of the non-mevalonate (MEP) pathway, described in Hintz *et al.* (2001), the chemical synthesis of HDMAPP has been achieved by a number of laboratories. *In vitro in vivo* pharmacodynamics of Vγ9/Vδ2 +T cell stimulation as elucidated by the inventors have shown that HDMAPP is surprisingly effective in activating γδ T cell activity, and may be administered to mammals in a low dose administration regimens. *in vitro* EC50 for HDMAPP the EC50 is about 0.6 nM, while the *in vivo* EC50 (in cynomolgus monkeys) for HDMAPP is about 5 pM (*in vivo* dosage studies described in PCT Publication No. WO 04/050096).

Preferred methods for the synthesis of HDMAPP are described in Example 1 and in Wolff et al., Tetrahedron Letters (2002) 43:2555 and Hecht et al., Tetrahedron Letters (2002) 43: 8929, teaching methods of preparing HDMAPP compounds.

A suitable amount of a compound of Formula I to III such as HDMAPP for augmenting or enhancing an immune response in a human is a dose that lies between about 1 μg/kg and about 20 mg/kg, preferably between about 10 μg/kg and about 2 mg/kg, more preferably between about 20 μg/kg and about 1 mg/kg, even more preferably between about 20 μg/kg and 100 μg/kg. In preferred exemplary compounds, a compound of formula XII to XVII, is administered in a dosage (single administration) between about 1 μg/kg and about 1 mg/kg, preferably in a low-dose administration regimen having dosage between about 10 μg/kg and 100 μg/kg.

*CHDMAPP*

The synthesis of CHDMAPP can be carried according to any suitable method. Examples include the methods of Nakamura *et al.* (1973), Zoretic and Zhang (1996) or Umbreit and Sharpless (1977), to produce a E-hydroxydimethylallyl type synthon prior to phosphorylation or phosphonation. Phosphorylation or phosphonation can then be carried out according to methods described in PCT patent publication No. WO 03/050128, Brondino *et al*., (1996), or Valentijn *et al*. (1991),

A suitable amount of a compound of Formula I to III such as CHDMAPP for augmenting or enhancing an immune response in a human is a dose that lies between about 1 μg/kg and about 20 mg/kg, preferably between about 10 μg/kg and about 2 mg/kg, more preferably between about 20 μg/kg and about 1 mg/kg, even more preferably between about 20 μg/kg and 100 μg/kg. Thus, in preferred exemplary compounds, a compound of formula I to III, is administered in a dosage (single administration) between about 1 μg/kg and about 1 mg/kg, preferably in a low-dose administration regimen having dosage between about 10 μg/kg and 100 μg/kg.

*Pharmaceutical Preparations*

Formulation of a preparation comprising the antigen and polynucleotides of the present invention, with or without addition of an adjuvant composition, or formulation of the compound of the invention can be carried out using standard pharmaceutical formulation chemistries and methodologies all of which are readily available to the ordinarily skilled artisan. Where appropriate the compound of the invention may also be formulated and administered as described below.

For example, compositions containing one or more antigens or one or more nucleic acid molecules (e. g., present in a plasmid or viral vector) can be combined with one or more pharmaceutically acceptable excipients or vehicles to provide a liquid preparation.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions can also contain additional agents such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminium monostearate and gelatin. These excipients, vehicles and auxiliary substances are generally pharmaceutical agents that do not if administered alone induce an immune response in the individual receiving the composition, and which may be administered without undue toxicity.

Pharmaceutically acceptable salts can also be included therein, for example, mineral acid salts such as hydrochlorides, hydrobromides, phosphates, sulfates, and the like; and the salts of organic acids such as acetates, propionates,

malonates, benzoates, and the like. It is also preferred, although not required, that the preparation will contain a pharmaceutically acceptable excipient that serves as a stabilizer, particularly for peptide, protein or other like molecules if they are to be included in the vaccine composition. Examples of suitable carriers that also act as stabilizers for peptides include, without limitation, pharmaceutical grades of dextrose, sucrose, lactose, trehalose, mannitol, sorbitol, inositol, dextran, and the like. Compounds of the invention can be administered with Ringer's lactate, for example.

Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, vehicles and auxiliary substances is available in REMINGTONS PHARMACEUTICAL SCIENCES (Mack Pub. Co., N. J. 199 1).

*Vaccine Dosage*

The amount of protein in each vaccine dose is selected as an amount which induces an immunoprotective response without significant, adverse side effects in typical vaccinees. Such amount will vary depending upon which specific immunogen is employed and how it is presented. An optimal amount for a particular vaccine can be ascertained by standard studies involving observation of appropriate immune responses in vaccinated subjects. Following an initial vaccination, subjects may receive one or several booster immunizations adequately spaced. Such a vaccine formulation may be applied to a mucosal surface of a mammal in either a priming or boosting vaccination regime; or alternatively be administered systemically, for example via the transdermal, subcutaneous or intramuscular routes.

The formulations of the present invention maybe used for both prophylactic and therapeutic purposes. Accordingly, there is provided the use of a combination of a compound of Formula I in the manufacture of a vaccine for the prophylaxis and the treatment of viral, bacterial, parasitic infections, allergy, cancer and other nonchronic disorders. Accordingly, the present invention provides for a method of treating a mammal susceptible to or suffering from an infectious disease or cancer, or allergy, or autoimmune disease. In a further aspect of the present invention there is provided a vaccine or adjuvant combination, comprising a compound of Formula I, as herein described for use as a medicament. Vaccine preparation is generally described in New Trends and Developments in Vaccines, edited by Voller et al., University Park Press, Baltimore, Maryland, U.S.A. 1978.

It is foreseen that compositions of the present invention will be used to formulate vaccines containing antigens derived from a wide variety of sources. For example, antigens as discussed in more detail herein typically include human, bacterial, or viral nucleic acid, pathogen derived antigen or antigenic preparations, tumor derived antigen or antigenic preparations, host-derived antigens, including peptides derived from IgE, such as the histamine releasing decapeptide of IgE, recombinantly produced protein or peptides, and chimeric fusion proteins.

There is provided by the present invention a systemic vaccine composition comprising an antigen and a compound of Formula I. Accordingly, there is provided a method of treatment of an individual susceptible to or suffering from a disease by the administration of a composition as substantially described herein through the systemic route of said individual. Also provided is a method to prevent an individual from contracting a disease selected from the group comprising infectious bacterial and viral diseases, parasitic diseases, prostate, breast, coiorectal, lung, pancreatic, renal, ovarian or melanoma cancers; non-cancer chronic disorders, allergy, Alzheimer, atherosclerosis, comprising the administration of a composition as substantially described herein through the systemic route of said individual.

Further aspects and advantages of the present invention will be disclosed in the following experimental section, which should be regarded as illustrative and not limiting the scope of the present application.

EXAMPLES

**EXAMPLE 1 - Synthesis of HDMAPP**

(E)-4-Hydroxy-3-methylbut-2-enyl diphosphate is prepared according to the method of Wolff et al., Tetrahedron Letters (2002) 43:2555 or Hecht et al., Tetrahedron Letters (2002) 43: 8929. For the purpose of performing biological testing, the aqueous solutions of the product are sterilized by filtration through a 0.2 $\mu$m filter and stored at -20 °C. In the case of testing performed *in vivo*, the solutions are passed beforehand through a DOWEX 50WX8-200 cationic resin column (sodium form) eluted by two column volumes of deionized water.

**EXAMPLE 2 - Synthesis of C-HDMAPP**

C-HDMAPP synthesis is carried out as follows, the scheme for which is also shown in Figure 1. References in Example 2 are made to Figure 1 by showing the reference number in brackets.

Preparation of (E)-4-chloro-2-methylbut-2-en-1-ol [1]:

Following the method of Hecht *et al.* (Hecht et al., Tetrahedron Letters, 43 (2002) 8929-8933) commercially available 2-methyl-2-vinyloxirane is converted into (E)-4-chloro-2-methylbut-2-en-1-ol [1] by treatment with TiCl4 at -80°C to -90 °C.

Preparation of (E)-4-chloro-2-methylbut-2-en-1-(pyranyl-2'-oxy) **[2]:**

Following the method of Miyashita *et al.* (Miyashita et al., J. Org. Chem. 42 (1977) 3772-3774), the allylic alcohol **[1]** is converted into a protected form **[2]** by reaction of **[1]** with Dihydropyrane (DHP) in the presence of Pyridinium p-Toluenesulfonate (PPTs).

Preparation of Methyl methylphosphonomorpholidate **[3]:**

Following the method of Valentijn *et al.* for the preparation of Farnesyl Pyrophosphate analogues (Valentijn et al., Synlett (1991) 663-664), the phosphonylating agent **[3]** is prepared by treatment of commercially available methylphosphonic dichloride with morpholine and methanol.

Preparation of intermediate [4]

Following the method of Valentijn *et al.* (Valentijn et al., Synlett (1991) 663-664), intermediate **[4]** is prepared by reaction of **[2]** with methyl lithiomethylphosphonomorpholidate obtained *in situ* from the phosphonylating agent **[3].**

Preparation of (E)-5-hydroxy-4-methylpent-3-enyl pyrophosphonate (C-HDMAPP)

A crude solution of C-HDMAPP is obtained in a 3 step procedure:

demethylation of intermediate **[4]** by treatment with tetra-n-butylammonium hydroxide in methanol as reported by Phan and Poulter (J. Org. Chem. (2001), 66, 6705-6710),
coupling with phosphoric acid following the procedure of Valentijn *et al.* (Valentijn et al., Synlett (1991) 663-664), and
deprotection of the pyranyl-2'-oxy group by subsequent treatment of the pyrophosphonate ester with chlorhydric acid at pH 1-2 to yield a crude solution of C-HDMAPP.

The crude salt of C-HDMAPP obtained at this stage is converted to the ammonium form by cation-exchange over DOWEX 50WX8-200 resin (ammonium form). Purification of the resulting solution is performed by chromatography over silica gel using 27 % ammonia solution/2-propanol 50/50 (v/v) as eluant. For the purpose of performing biological testing, the aqueous solutions of the product are sterilized by filtration through a 0.2 μm filter and stored at -20 °C. In the case of testing performed *in vivo,* the solutions are passed beforehand through a DOWEX 50WX8-200 cationic resin column (sodium form) eluted by two column volumes of deionized water.

**EXAMPLE *3 - In Vitro* and *In Vivo* Dosage Response for HDMAPP Compound**

*Cytokine release assay*

Cells (primary polyclonal human Vγ9/Vδ2 T cells which had been expanded *in vitro* and stored frozen at day 12-15 of expansion) are thawed and rinsed them twice and centrifuged. Upon elimination of supernatant and resuspension of cells, cells are incubated for 24h at 37°C in the presence of IL2 100 UI/ml (final concentration). Cells are washed and centrifuged, following which the supernatant is eliminated and the cells are resuspended and adjusted to the adequate final concentration. Cells are added to the wells of a 96-well plate.

To one row of wells are added a standard diluation series of 3-(bromomethyl)-3-butanol-1-yl-diphosphate (BrHPP). Compounds to be tested, in this case CHDMAPP and HDMAPP are added to experimental wells, after several dilutions.

Full plates are incubated 24 hours at 37°C for stimulation of the γδ cells with the BrHPP standard as well as isopentenyl pyrophosphate (IPP) for comparison, and test compounds CHDMAPP and HDMAPP. After this time, 100 μl of culture supernatant is taken for TNFα dosage. Measurement of the released TNFa dosageis performed as described by the manufacturer's instruction in the TNFa enzyme immunoassay kit (ref. 11121, Immunotech - Beckman Coulter). OD at 405nm is read, the OD being proportional to the concentration of released TNFα in the culture supernatant. The data are processed with the Excel software to compare concentration of test compound versus concentration of TNFα

and for the calculation of the EC50 for each test compound.

*HDMAPP and CHDMAPP in vitro and in vivo bioactivity*

The bioactivity of the compounds HDMAPP and CHDMAPP was assessed using a TNFα release assay. *In vitro* activity is shown in Figure 2. For purposes of comparison, the compounds isopentenyl pyrophosphate (IPP) and bromohydrin pyrophosphate (BrHPP) were included in the *in vivo* comparison. The *in vitro* EC50 for HDMAPP was determined to be about 0.758 nM while the *in vitro* EC50 for CDMAPP is about 0.873 nM. By contrast, the less potent BrHPP and IPP showed an EC50 value of about 18.59 nM and 30.98 μM, respectively.

**EXAMPLE 4 - Adjuvant Effect in Humoral and Cell-Mediated Immune Response**

The systemic humoral and cell-mediated immune responses are examined in cynomolgus monkeys vaccinated with an HIV protein co-formulated with HDMAPP or CHDMAPP compound.

The purpose of these experiments is to determine if immunization with a recombinant HIV protein, Gp120, Gp160, Gag, Rev, Nef or Tat protein, formulated with HDMAPP or CHDMAPP can elicit functional serum antibody titers that are greater than those achieved after immunization with HIV protein alone. In the experiments native gp120, gp160, gag or Tat protein is obtained (BD Biosciences, USA or as described in Novitsky V, et al. J. Virol. 75: 9210, 2001). The protein purity is estimated by SDS-PAGE and antigen capture ELISA. CHDMAPP or HDMAPP are prepared according to Examples 1 and 2 respectively.

Cynomolgus monkeys are vaccinated intramuscularly on weeks 0, 4 and optionally again at week 8 with the recombinant HIV protein at a dosage of between 5 and 20 μg/dose.

The vaccines are prepared such that HIV-derived protein is co-formulated with a dose of HDMAPP or CHDMAPP that lies between about 1 μg/kg and about 20 mg/kg or preferably between about 20 μg/kg and 100 μg/kg. Control monkeys are injected with HIV protein in PBS alone.

Blood samples are taken 2 weeks after the immunization (*e.g.* at week 6 and week 10). Anti-HIV protein antibody response is measured using standard ELISA-based assays to detect anti-HIV protein antibody levels.

The cell mediated response is detected using assays to measure the frequency of cytokine-secreting cells in blood samples. This can be carried out using any suitable assay such as an EliSpot assays (Mabtech, Sweden) to detect IFN-gamma-producing cells as described in Derby et al., Cytokine. 2001 Jan 21;13(2):85-90 or assays as described in Novitsky V, et al. Identification of human immunodeficiency virus type 1 subtype C Gag-, Tat-, Rev-, and Nef-specific ELISpot-based cytotoxic T-lymphocyte responses for AIDS vaccine design. J. Virol. 75: 9210, 2001.

**EXAMPLE 5 - Adjuvant effect of a phosphoantigen on a HIV vaccine and induction of mucosal immunity**

Animals will be primed with VSV-gag (3 injections, 1 week apart) and boosted one (1) month later with Adeno-gag (3 injections, 1 week apart). VSV-gag vaccine is described for example in Haglund et al., J Virol. 2002; 76(15): 7506-17, and Adeno-gag vaccine is described for example in Zhong et al., Eur J Immunol. 2000 Nov;30(11):3281-90. Vaccination formulations will contain BrHpp (an exemplary phosphoantigen compound) and will be injected i.v. at a saturating dose. Phenotypic and functional changes in each conventional antigen-specific cell population will be investigated. To confirm activation of mucosal immunity at uro-genital (or other mucosal) sites after administration of the vaccine compositions via the nasal mucosa, locally-induced cytokines will be measured at chosen mucosal sited. Female animals can be used to assess the activation of mucosal immunity uro-genital sites.

*Study plan*:

- group I: control with vehicle alone (4 animals, including 2 females)
- group II: Phosphoantigen compound + IL2 (control 2, 4 animals)
- group III: Phosphoantigen compound alone (6 animals)
- group IV: vaccine alone (6 animals)
- group V: Phosphoantigen compound + vaccine (6 animals)

**EXAMPLE 6 - Phosphoantigen-specific VγV82 T Cells and Degree of the Vaccine-induced Protection Against *M. tuberculosis***

Experimental design: Phosphoantigens can also be used to induce immunity to bacterial pathogens, such as

*M. tuberculosis.* Using a recently validated monkey tuberculosis model, a total of sixteen cynomolgus monkeys (Macaca mulatta) are divided into two groups, experimental and control groups. The experimental group is immunized with a vaccine composition containing phosphoantigen and *M. tuberculosis.* The control group is vaccinated similarly with a composition containing glucose and *M. tuberculosis,* according to standard vaccinations protocols, for example a first prime dose and a boost dose 5 weeks later. T-cell immune responses of Vγ9Vδ2 T cells are assessed biweekly after immunization. Four months after immunization, monkeys are challenged with 100 CFU of *M. tuberculosis* by aerosol as previously described (Shen *et al.*, 2002). Following *M. tuberculosis* challenge, monkeys are followed clinically, pathologically, and immunologically. Blood sampling and bronchioalveolar lavage is done biweekly after *M. tuberculosis* challenge. Three months after *M. tuberculosis* challenge, monkeys are sacrificed for necropsy and histology studies.

In order to assess the role of Vγ9Vδ2 T-cells and their role in immunity against tuberculosis, a total of 16 monkeys are divided into two groups, experimental and control groups. Animals are immunized with compositions containing phospohantigen and *M. tuberculosis* (experimental group) or glucose and *M. tuberculosis* (control group). The experimental group is treated with anti-Vd2 antibody for depletion of Vγ9Vδ2 T cells and challenged with 100 CFU of *M. tuberculosis* by aerosol as previously described (Shen *et al.*, 2002) immediately after Vγ9Vδ2 T-cell depletion. Following *M. tuberculosis* challenge, monkeys are followed clinically, pathologically, and immunologically. Blood sampling and bronchioalveolar lavage is done biweekly after *M. tuberculosis* challenge. Three months after *M. tuberculosis* challenge, monkeys are sacrificed for necropsy and histology studies.

**EXAMPLE 7 - Tuberculosis vaccination in nonhuman primates using "Hybrid I' Antigen in combination with Phosphoantigen**

The 'Hybrid I' vaccine is a recombinant protein fusing 2 dominant epitopes called Ag85B and EZ6 from BCG ( Weinrich Olsen A, van Pinxteren LA, Meng Okkels L, Birk Rasmussen P and Andersen P (2001) Protection of mice with a tuberculosis vaccine based on a fusion protein of Ag 85b and ESAT-6. Infection and Immunity 69(5), pp2773-2778 - Olsen AW, Williams A, Okkels LM, Hatch G and Andersen P (2004) Protective effect of a tuberculosis subunit vaccine based on a fusion of antigen 85b and ESAT-6 in the aerosol guinea pig model. Infection and Immunity 72(10), pp6148-6150). It is distributed by the TB Vaccine Cluster (Unité de Génétique Mycobactérienne, Institut Pasteur, France) network in injectable form. Hybrid I is formulated in SSI adjuvant (available from Statens Serum Institut, Copenhagen, Denmark).

Sixteen monkeys were divided into the following groups:

Group I: Hybrid I formulation only (8 individuals)
Group II: Hybrid I formulation in combination with phosphoantigen* (8 individuals)

Phosphoantigen, in this example (E)-5-hydroxy-4-methylpent-3-enyl pyrophosphonate (CHDMAPP) according to Formulas I and V herein, was administered to the monkeys at a dose of about 2 mg/kg. Phosphoantigen compound and antigen was injected 3 times according to the following schedule: a prime dose, a first boost dose 4 weeks after the prime dose, and then second boost dose on week 13), with the Phosphoantigen compound being administered intramuscularly (i.m.) conjointly with the antigen (Hybrid I), but in a separate formulation.

Follow-up of the vaccine response was carried out the week following the first boost dose, the week following the second boost dose, and 4 weeks after the second boost dose. Follow-up comprised determining the serum dosage/ level of various cytokines, including IFNγ, TNF-alpha, IL2, IL4, IL5 and IL6. Results of the follow-up are shown in Figure 3. Addition of the Phosphoantigen compound to the Hybrid-I formulation produced a large and statistically significant increase in cytokine production over that observed with the Hybrid-I formulation alone. There was also a large increase of cytokine production for those cytokines known to be induced by phosphoantigen alone (IFNγ) over that observed with the phosphoantigen alone. The results suggest that administration of a compound of Formula I conjointly with an antigen is capable of eliciting the production of a number of lymphocyte growth factors involved in the proliferative capacity and induction of T cell activity as well as the recruitment and activation of cell populations such as dendritic cells and macrophages, in turn indicative of an ability to provide a protective response when used in combination with a vaccine.

Alternatively or in addition, the proportion of INFγ-expressing cells can be determined, as analyzed by Elispot. Other tests, such as, phenotype analysis for different lymphocyte populations, IL-2 level in serum, and delayed-type hypersensitivity (DTH) to i.d. Hybrid-I or tuberculin and *in vitro* lymphoproliferation to different antigens can also be performed to analyze the immune response of the animals.

## Claims

**1.** A vaccine adjuvant composition comprising a γδT cell activator of Formula I:

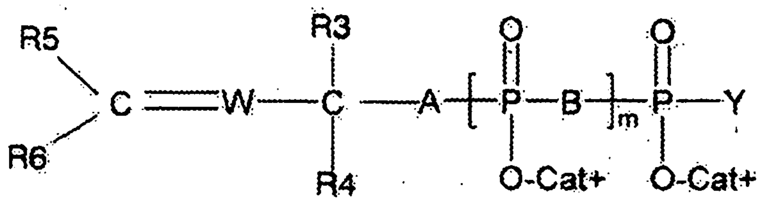

(I)

in which $R_3$, $R_4$, and $R_5$, identical or different, are a hydrogen or $(C_1\text{-}C_3)$alkyl group, W is -CH- or-N-, $R_6$ is an $(C_2\text{-}C_3)$ acyl, an aldehyde, an $(C_1\text{-}C_3)$alcohol, or an $(C_2\text{-}C_3)$ester, Cat+ represents one (or several, identical or different) organic or mineral cation(s) (including the proton), B is O or NH, m is an integer from 1 to 3, A is O, NH, CHF, $CF_2$ or $CH_2$, and Y is $O^-$Cat+, a nucleoside, or a radical -A-R, wherein R is a linear, branched, or cyclic, aromatic or not, saturated or unsaturated, $C_1\text{-}C_{50}$ hydrocarbon group, optionally interrupted by at least one heteroatom, wherein said hydrocarbon group comprises an alkyl, an alkylcnyl, or an alkynyl, preferably an alkyl or an alkylene, which can be substituted by one or several substituents selected from the group consisting of : an $(C_1\text{-}C_6)$alkyl, an $(C_2\text{-}C_6)$ alkylenyl, an $(C_2\text{-}C_6)$alkynyl, an $(C_2\text{-}C_6)$epoxyalkyl, an aryl, an heterocycle, an $(C_1\text{-}C_6)$alkoxy, an $(C_2\text{-}C_6)$acyl, an $(C_1\text{-}C_6)$alcohol, a carboxylic group (-COOH), an $(C_2\text{-}C_6)$ester, an $(C_1\text{-}C_6)$amine, an amino group ($-NH_2$), an amide ($-CONH_2$), an $(C_1\text{-}C_6)$imine, a nitrile, an hydroxyl (-OH), a aldehyde group (-CHO), an halogen, an $(C_1\text{-}C_6)$halogenoalkyl, a thiol (-SH), a $(C_1\text{-}C_6)$thioalkyl, a $(C_1\text{-}C_6)$sulfone, a $(C_1\text{-}C_6)$sulfoxide, and a combination thereof, wherein said γδT cell activator is present in amount sufficient to elicit a CTL response in a mammal.

**2.** A vaccine composition comprising an antigen or a combination of antigens, and a γδT cell activator of Formula I:

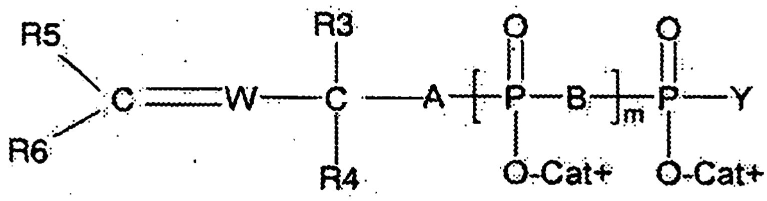

(I)

in which $R_3$, $R_4$, and $R_5$ , identical or different, are a hydrogen or $(C_1\text{-}C_3)$alkyl group, W is -CH- or - N-, $R_6$ is an $(C_2\text{-}C_3)$acyl, an aldehyde, an $(C_1\text{-}C_3)$alcohol, or an $(C_2\text{-}C_3)$ester; Cat+ represents one (or several, identical or different) organic or mineral cation(s) (including the proton), B is O or NH, m is an integer from 1 to 3, A is O, NH, CHF, $CF_2$ or $CH_2$, and Y is $O^-$Cat+, a nucleoside, or a radical -A-R, wherein R is a linear, branched, or cyclic, aromatic or not, saturated or unsaturated, $C_1\text{-}C_{50}$ hydrocarbon group, optionally interrupted by at least one heteroatom, wherein said hydrocarbon group comprises an alkyl, an alkylenyl, or an alkynyl, preferably an alkyl or an alkylene, which can be substituted by one or several substituents selected from the group consisting of : an $(C_1\text{-}C_6)$alkyl, an $(C_2\text{-}C_6)$alkylenyl, an $(C_2\text{-}C_6)$alkynyl, an $(C_2\text{-}C_6)$epoxyalkyl, an aryl, an heterocycle, an $(C_1\text{-}C_6)$alkoxy, an $(C_2\text{-}C_6)$acyl, an $(C_1\text{-}C_6)$alcohol, a carboxylic group (-COOH), an $(C_2\text{-}C\text{-}_6)$ester, an $(C_1\text{-}C_6)$amine, an amino group ($-NH_2$), an amide ($-CONH_2$), an $(C_1\text{-}C_6)$imine, a nitrile, an hydroxyl (-OH), a aldehyde group (-CHO), an halogen, an $(C_1\text{-}C_6)$halogenoalkyl, a thiol (-SH), a $(C_1\text{-}C_6)$thioalkyl, a $(C_1\text{-}C_6)$sulfone, a $(C_1\text{-}C_4)$sulfoxide, and a combination thereof, wherein said γδT cell activator is present in amount sufficient to elicit a CTL response in a mammal.

**3.** The composition according to claim 1 or 2, wherein said γδT cell activator is a compound of formula (II) or (III):

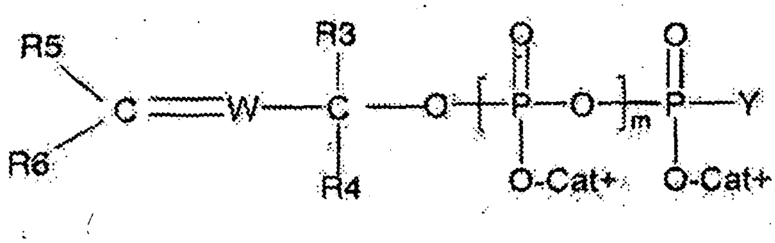
(II)

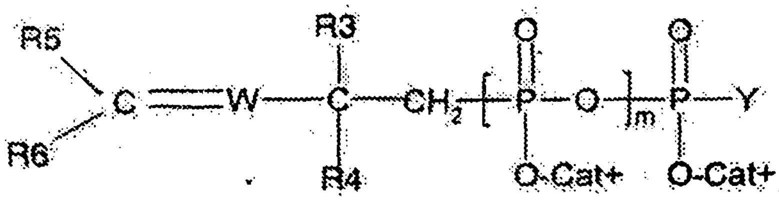
(III)

wherein $R_3$, $R_4$, and $R_5$, identical or different, are a hydrogen or $(C_1-C_3)$alkyl group, W is CH- or -N-, $R_6$ is an $(C_2-C_3)$ acyl, an aldehyde, an $(C_1-C_3)$alcohol, or an $(C_2-C_3)$ester, Cat+ represents one (or several, identical or different) organic or mineral cation(s) (including the proton), m is an integer from 1 to 3, and Y is $O^-Cat+$ or a nucleoside.

4. The vaccine adjuvant composition according to claim 3, wherein said γδT cell activator is (E)-4-hydroxy-3-methyl-2-butenyl pyrophosphate (HDMAPP).

5. The vaccine adjuvant composition according to claim 3, wherein said γδT cell activator is (E)-5-hydroxy-4-methylpent-3-enyl pyrophosphonate (CHDMAPP).

6. The vaccine adjuvant composition according to claim 3 wherein said γδT cell activator is (E)-5-hydroxy-4-methylpent-3-enyl aminophosphate (NHDMAPP).

7. The vaccine composition according to claim 2-6, wherein said vaccine composition prevents or treats a microbial infection or prevents or treats a tumor.

8. The composition according to any one of claim 1-7, wherein said γδT cell activator is present in amount sufficient to elicit a CTL response enhanced in comparison to that obtained with by administering the vaccine or composition comprising an antigen in the absence of said γδT cell activator.

9. The composition according to any one of claims 1 to 8 comprising between about 50 μg and about 100 mg of said γδ T cell activator.

10. The composition according to any one of claims 1 to 8 comprising between about 500 μg and about 10 mg of said γδ T cell activator.

11. The composition according to any one of claims 1 to 8 comprising less than about 100 mg of said γδ T cell activator.

12. A composition according to any one of claims 1 to 8 comprising less than about 10 mg of said γδ T cell activator.

13. The composition according to any one of claims 1 to 8 comprising less than about 1 mg of said γδ T cell activator.

14. Use of a vaccine composition according to any one of claims 2-13 for preparing a composition for improving the potency of a vaccine in a subject, or for treating a disease or for protecting a subject from a disease, more particularly a tumor disease or a microbial infection disease.

15. The use according to claim 14, wherein said γδT cell activator and said antigen(s) are administered conjointly.

16. A vaccine kit comprising a suitable container containing a vaccine composition according to any one of claims 2-13.

**17.** A vaccine kit comprising a suitable container containing a vaccine adjuvant composition according to any one of claims 1, 3-6 and 8-13 and a suitable container containing an antigen or a combination of antigens.

**18.** The use of any of claims 14-17, wherein said therapeutically effective amount of a) an antigen or a said combination of antigens and (b) a γδ T cell activator is aiming to be administered to said subject repeatedly after an interval of at least 1 week following the previous administration of said antigen or a combination of antigens.

**19.** The use of any one of claims 14-17, wherein said therapeutically effective amount of (a) an antigen or said combination of antigens and (b) a γδ T cell activator is aiming to be administered repeatedly after an interval of at least 2 weeks following the previous administration of said antigen or a combination of antigens.

**Patentansprüche**

**1.** Eine Impfstoffadjuvans-Zusammensetzung, umfassend einen γδ-T-Zellaktivator der Formel I:

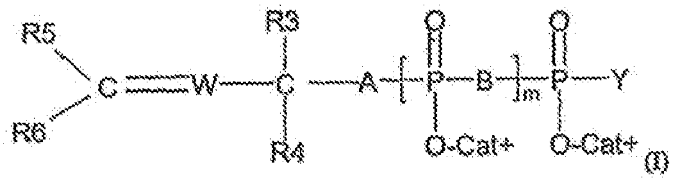

in der $R_3$, $R_4$ und $R_5$ identisch oder verschieden sind und ein Wasserstoff oder eine ($C_1$-$C_3$)-Alkylgruppe sind, W -CH- oder -N- ist, $R_6$ ein ($C_2$-$C_3$)-Acyl, ein Aldehyd, ein ($C_1$-$C_3$)-Alkohol oder ein ($C_2$-$C_3$)-Ester ist, Cat+ für ein (oder mehrere identische oder verschiedene) organisches oder mineralisches Kation(en) (einschließlich Proton) steht, B O oder NH ist, m eine ganze Zahl von 1 bis 3 ist, A O, NH, CHF, $CF_2$ oder $CH_2$ ist und Y $O^-$Cat+, ein Nukleosid oder ein Rest -AR ist, worin R eine lineare, verzweigte oder cyclische, aromatische oder nicht-aromatische, gesättigte oder ungesättigte, $C_1$-$C_{50}$-Kohlenwasserstoffgruppe ist, die gegebenenfalls von wenigstens einem Heteroatom unterbrochen ist, wobei besagte Kohlenwasserstoffgruppe ein Alkyl, ein Alkylenyl oder ein Alkenyl, vorzugsweise ein Alkyl oder ein Alkylen, umfasst, die mit einem oder mehreren Substituenten substituiert sein kann, der aus der Gruppe ausgewählt ist, bestehend aus: einem ($C_1$-$C_6$)-Alkyl, einem ($C_2$-$C_6$)-Alkylenyl, einem ($C_2$-$C_6$)-Alkinyl, einem ($C_2$-$C_6$)-Epoxyalkyl, einem Aryl, einem Heterocyclus, einem ($C_1$-$C_6$)-Alkoxy, einem ($C_2$-$C_6$)-Acyl, einem ($C_1$-$C_6$)-Alkohol, einer Carboxylgruppe (-COOH), einem ($C_2$-$C_6$)-Ester, einem ($C_1$-$C_6$)-Amin, einer Aminogruppe (-$NH_2$), einem Amid (-$CONH_2$), einem ($C_1$-$C_6$)-Imin, einem Nitril, einem Hydroxyl (-OH), einer Aldehydgruppe (-CHO), einem Halogen, einem ($C_1$-$C_6$)-Halogenalkyl, einem Thiol (-SH), einem ($C_1$-$C_6$)-Thioalkyl, einem ($C_1$-$C_6$)-Sulfon, einem ($C_1$-$C_6$)-Sulfoxid und einer Kombination davon, worin besagter γδ-T-Zellaktivator in einer hinreichenden Menge vorliegt, um eine CTL-Antwort in einem Säuger hervorzurufen.

**2.** Eine Impfstoffzusammensetzung, umfassend ein Antigen oder eine Kombination von Antigenen und einen γδ-T-Zellaktivator der Formel I:

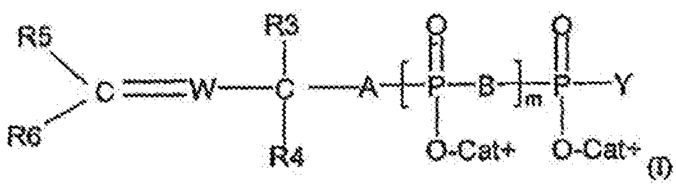

in der $R_3$, $R_4$ und $R_5$ identisch oder verschieden sind und ein Wasserstoff oder eine ($C_1$-$C_3$)-Alkylgruppe sind, W -CH- oder -N- ist, $R_6$ ein ($C_2$-$C_3$)-Acyl, ein Aldehyd, ein ($C_1$-$C_3$)-Alkohol oder ein ($C_2$-$C_3$)-Ester ist, Cat+ für ein (oder mehrere identische oder verschiedene) organisches oder mineralisches Kation(en) (einschließlich Proton) steht, B O oder NH ist, m eine ganze Zahl von 1 bis 3 ist, A O, NH, CHF, $CF_2$ oder $CH_2$ ist und Y $O^-$Cat+, ein Nukleosid oder ein Rest -AR ist, worin R eine lineare, verzweigte oder cyclische, aromatische oder nicht-aromatische, gesättigte oder ungesättigte, $C_1$-$C_{50}$-Kohlenwasserstoffgruppe ist, die gegebenenfalls von wenigstens einem Heteroatom unterbrochen ist, wobei besagte Kohlenwasserstoffgruppe ein Alkyl, ein Alkylenyl oder ein Alkinyl, vorzugsweise ein

Alkyl oder ein Alkylen, umfasst, die mit einem oder mehreren Substituenten substituiert sein kann, der aus der Gruppe ausgewählt ist, bestehend aus: einem ($C_1$-$C_6$)-Alkyl, einem ($C_2$-$C_6$)-Alkylenyl, einem ($C_2$-$C_6$)-Alkinyl, einem ($C_2$-$C_6$)-Epoxyalkyl, einem Aryl, einem Heterocyclus, einem ($C_1$-$C_6$)-Alkoxy, einem ($C_2$-$C_6$)-Acyl, einem ($C_1$-$C_6$)-Alkohol, einer Carboxylgruppe (-COOH), einem ($C_2$-$C_6$)-Ester, einem ($C_1$-$C_6$)-Amin, einer Aminogruppe (-$NH_2$), einem Amid (-$CONH_2$), einem ($C_1$-$C_6$)-Imin, einem Nitril, einem Hydroxyl (-OH), einer Aldehydgruppe (-CHO), einem Halogen, einem ($C_1$-$C_6$)-Halogenalkyl, einem Thiol (-SH), einem ($C_1$-$C_6$)-Thioalkyl, einem ($C_1$-$C_6$)-Sulfon, einem ($C_1$-$C_6$)-Sulfoxid und einer Kombination davon, worin besagter γδ-T-Zellaktivator in einer hinreichenden Menge vorliegt, um eine CTL-Antwort in einem Säuger hervorzurufen.

**3.** Die Zusammensetzung gemäß Anspruch 1 oder 2, worin besagter γδ-T-Zellaktivator eine Verbindung der Formel (II) oder (III) ist:

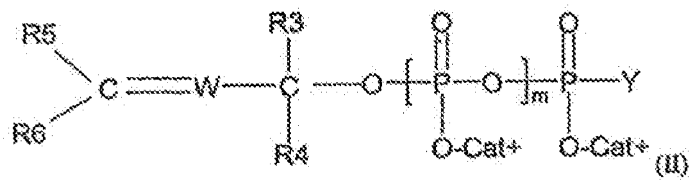

(II)

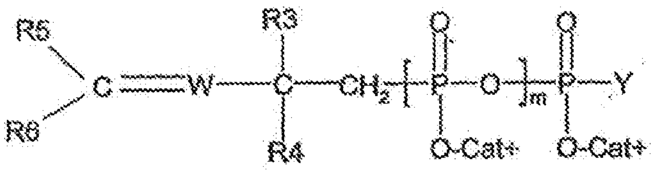

(III)

worin $R_3$, $R_4$ und $R_5$ identisch oder verschieden sind und ein Wasserstoff oder eine ($C_1$-$C_3$)-Alkylgruppe sind, W -CH- oder -N- ist, $R_6$ ein ($C_2$-$C_3$)-Acyl, ein Aldehyd, ein ($C_1$-$C_3$)-Alkohol oder ein ($C_2$-$C_3$)-Ester ist, Cat+ für ein (oder mehrere identische oder verschiedene) organisches oder mineralisches Kation(en) (einschließlich Proton) steht, m eine ganze Zahl von 1 bis 3 ist, und Y $O^-$Cat+ oder ein Nukleosid ist.

**4.** Die Impfstoffadjuvans-Zusammensetzung gemäß Anspruch 3, worin besagter γδ-T-Zellaktivator (E)-4-Hydroxy-3-methyl-2-butenylpyrophosphat (HDMAPP) ist.

**5.** Die Impfstoffadjuvans-Zusammensetzung gemäß Anspruch 3, worin besagter γδ-T-Zellaktivator (E)-5-Hydroxy-4-methylpent-3-enylpyrophosphonat (CHDMAPP) ist.

**6.** Die Impfstoffadjuvans-Zusammensetzung gemäß Anspruch 3, worin besagter γδ-T-Zellaktivator (E)-5-Hydroxy-4-methylpent-3-enylaminophosphat (NHDMAPP) ist.

**7.** Die Impfstoffzusammensetzung gemäß Ansprüchen 2 bis 6, worin besagte Impfstoffzusammensetzung eine mikrobielle Infektion verhindert oder behandelt oder einen Tumor verhindert oder behandelt.

**8.** Die Zusammensetzung gemäß einem der Ansprüche 1 bis 7, worin besagter γδ-T-Zellaktivator in einer hinreichenden Menge vorliegt, um eine CTL-Antwort hervorzurufen, die verglichen mit derjenigen Antwort verstärkt ist, die mit einer Verabreichung des Impfstoffes oder der ein Antigen umfassenden Zusammensetzung in Abwesenheit besagten γδ-T-Zellaktivators erhalten wird.

**9.** Die Zusammensetzung gemäß einem der Ansprüche 1 bis 8, umfassend zwischen etwa 50 µg und etwa 100 mg besagten γδ-T-Zellaktivator.

**10.** Die Zusammensetzung gemäß einem der Ansprüche 1 bis 8, umfassend zwischen etwa 500 µg und etwa 10 mg besagten γδ-T-Zellaktivator.

**11.** Die Zusammensetzung gemäß einem der Ansprüche 1 bis 8, umfassend weniger als etwa 100 mg besagten γδ-T-Zellaktivator.

12. Eine Zusammensetzung gemäß einem der Ansprüche 1 bis 8, umfassend weniger als etwa 10 mg besagten γδ-T-Zellaktivator.

13. Die Zusammensetzung gemäß einem der Ansprüche 1 bis 8, umfassend weniger als etwa 1 mg besagten γδ-T-Zellaktivator.

14. Verwendung einer Impfstoffzusammensetzung gemäß einem der Ansprüche 2 bis 13 zur Zubereitung einer Zusammensetzung zur Verbesserung der Wirksamkeit eines Impfstoffes in einer Person oder zur Behandlung einer Krankheit oder zum Schutz einer Person vor einer Erkrankung, insbesondere einer Tumorerkrankung oder einer mikrobiellen Infektionskrankheit.

15. Die Verwendung gemäß Anspruch 14, worin besagter γδ-T-Zellaktivator und besagtes Antigen(e) gemeinsam verabreicht werden.

16. Ein Impfstoff-Kit, umfassend einen geeigneten Behälter, der eine Impfstoffzusammensetzung gemäß einem der Ansprüche 2 bis 13 enthält.

17. Ein Impfsto-ff-Kit, umfassend einen geeigneten Behälter, der eine Impfstoffadjuvans-Zusammensetzung gemäß einem der Ansprüche 1, 3 bis 6 und 8 bis 13 enthält, und einen geeigneten Behälter, der ein Antigen oder eine Kombination von Antigenen enthält.

18. Die Verwendung nach einem der Ansprüche 14 bis 17, worin besagte therapeutisch wirksame Menge a) eines Antigens oder einer besagten Kombination von Antigenen und (b) eines γδ-T-Zellaktivators an besagte Person in einem Abstand von wenigstens 1 Woche nach der vorhergehenden Verabreichung besagten Antigens oder einer Kombination von Antigenen wiederholt verabreicht werden soll.

19. Die Verwendung nach einem der Ansprüche 14 bis 17, worin besagte therapeutisch wirksame Menge a) eines Antigens oder einer besagten Kombination von Antigenen und (b) eines γδ-T-Zellaktivators an besagte Person in einem Abstand von wenigstens 2 Wochen nach der vorhergehenden Verabreichung besagten Antigens oder einer Kombination von Antigenen wiederholt verabreicht werden soll.

## Revendications

1. Composition d'adjuvant de vaccin comprenant un activateur de cellules γδT de Formule 1:

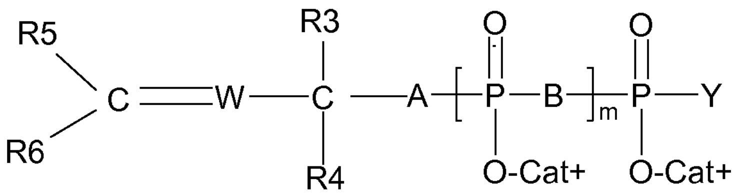

(I)

dans laquelle $R_3$, $R_4$, et $R_5$, identiques or différents, sont un hydrogène ou un groupe alkyle en ($C_1$-$C_3$), W est -CH- ou -N-, $R_6$ est un acyle en ($C_2$-$C_3$), un aldéhyde, un alcool en ($C_1$-$C_3$), ou un ester en ($C_2$-$C_3$), Cat+ représente un (ou plusieurs, identiques ou différents) cation(s) organique(s) ou minéral(s) (incluant le proton), B est O ou NH, m est un entier de 1 à 3, A est O, NH, CHF, $CF_2$ ou $CH_2$, et Y est $O^-$Cat+, un nucléoside, ou un radical -A-R, dans lequel R est un groupe hydrocarboné en $C_1$-$C_{50}$ linéaire, branché, ou cyclique, aromatique ou non, saturé ou insaturé, facultativement interrompu par au moins un hétéroatome, lequel groupe hydrocarboné comprend un alkyle, un alcényle, ou un alcynyle, de préférence un alkyle ou un alcényle, qui peut être substitué par un ou plusieurs substituants sélectionnés parmi le groupe constitué par : un alkyle en ($C_1$-$C_6$), un alcényle en ($C_2$-$C_6$), un alcynyle en ($C_2$-$C_6$), un époxyalkyle en ($C_2$-$C_6$), un aryle, un hétérocycle, un alkoxy en ($C_1$-$C_6$), un acyle en ($C_2$-$C_6$), un alcool en ($C_1$-$C_6$), un groupe carboxylique (-COOH), un ester en ($C_2$-$C_6$), une amine en ($C_1$-$C_6$), un groupe amino (-$NH_2$), une amide (-$CONH_2$), une imine en ($C_1$-$C_6$), un nitrile, un hydroxyl (-OH), un groupe aldéhyde (-CHO), un halogène,

un halogènoalkyle en ($C_1$-$C_6$), un thiol (-SH), un thioalkyle en ($C_1$-$C_6$), un sulfone en ($C_1$-$C_6$), un sulfoxyde en ($C_1$-$C_6$), et une combinaison de ceux-ci, dans laquelle ledit activateur de cellules γδT est présent en une quantité suffisante pour provoquer une réponse CTL chez un mammifère.

**2.** Composition vaccinale comprenant un antigène ou une combinaison d'antigènes, et un activateur de cellules γδT de Formule I:

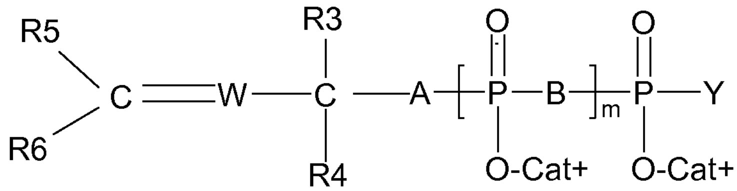
(I)

dans laquelle $R_3$, $R_4$, et $R_5$ , identiques or différents, sont un hydrogène ou un groupe alkyle en ($C_1$-$C_3$), W est -CH- ou -N-, $R_6$ est un acyle en ($C_2$-$C_3$), un aldéhyde, un alcool en ($C_1$-$C_3$), ou un ester en ($C_2$-$C_3$), Cat+ représente un (ou plusieurs, identiques ou différents) cation(s) organique(s) ou minéral(s) (incluant le proton), B est O ou NH, m est un entier de 1 à 3, A est O, NH, CHF, $CF_2$ ou $CH_2$, et Y est $O^-$Cat+, un nucléoside, ou un radical -A-R, dans lequel R est un groupe hydrocarboné en $C_1$-$C_{50}$ linéaire, branché, ou cyclique, aromatique ou non, saturé ou insaturé, facultativement interrompu par au moins un hétéroatome, lequel groupe hydrocarboné comprend un alkyle, un alcényle, ou un alcynyle, de préférence un alkyle ou un alcényle, qui peut être substitué par un ou plusieurs substituants sélectionnés parmi le groupe constitué par : un alkyle en ($C_1$-$C_6$), un alcényle en ($C_2$-$C_6$), un alcynyle en ($C_2$-$C_6$), un époxyalkyle en ($C_2$-$C_6$), un aryle, un hétérocycle, un alkoxy en ($C_1$-$C_6$), un acyle en ($C_2$-$C_6$), un alcool en ($C_1$-$C_6$), un groupe carboxylique (-COOH), un ester en ($C_2$-$C_6$), une amine en ($C_1$-$C_6$), un groupe amino (-$NH_2$), une amide (-$CONH_2$), une imine en ($C_1$-$C_6$), un nitrile, un hydroxyl (-OH), un groupe aldéhyde (-CHO), un halogène, un halogènoalkyle en ($C_1$-$C_6$), un thiol (-SH), un thioalkyle en ($C_1$-$C_6$), un sulfone en ($C_1$-$C_6$), un sulfoxyde en ($C_1$-$C_6$), et une combinaison de ceux-ci, dans laquelle ledit activateur de cellules γδT est présent en une quantité suffisante pour provoquer une réponse CTL chez un mammifère.

**3.** Composition selon la revendication 1 ou 2, dans laquelle ledit activateur de cellules γδT est un composé de formule (II) ou (III):

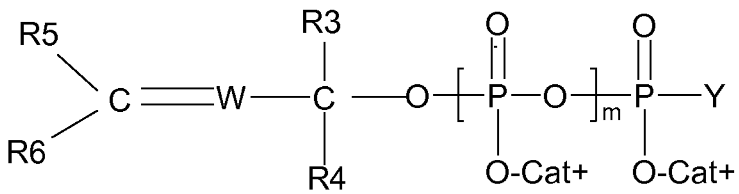
(II)

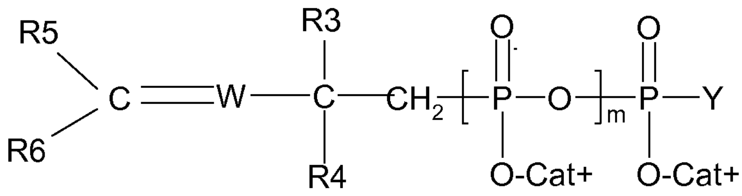
(III)

dans laquelle $R_3$, $R_4$, et $R_5$, identiques or différents, sont un hydrogène ou un groupe alkyle en ($C_1$-$C_3$), W est -CH- ou -N-, $R_6$ est un acyle en ($C_2$-$C_3$), un aldéhyde, un alcool en ($C_1$-$C_3$), ou un ester en ($C_2$-$C_3$), Cat+ représente un (ou plusieurs, identiques ou différents) cation(s) organique(s) ou minéral(s) (incluant le proton), m est un entier de 1 à 3, et Y est $O^-$Cat+ ou un nucléoside.

**4.** Composition d'adjuvant de vaccin selon la revendication 3, dans laquelle ledit activateur de cellules γδT est le (E)-4-hydroxy-3-méthyl-2-butènyl pyrophosphate (HDMAPP).

**5.** Composition d'adjuvant de vaccin selon la revendication 3, dans laquelle ledit activateur de cellules γδT est le (E)-5-hydroxy-4-méthylpent-3-ènyl pyrophosphonate (CHDMAPP).

**6.** Composition d'adjuvant de vaccin selon la revendication 3, dans laquelle ledit activateur de cellules γδT est le (E)-5-hydroxy-4-méthylpent-3-ènyl aminophosphate (NHDMAPP).

**7.** Composition vaccinale selon l'une quelconque des revendications 2-6, dans laquelle ladite composition vaccinale prévient ou traite une infection microbienne ou prévient ou traite une tumeur.

**8.** Composition selon l'une quelconque des revendications 1-7, dans laquelle ledit activateur de cellules γδT est présent en une quantité suffisante pour provoquer une réponse CTL augmentée en comparaison de celle obtenue en administrant un vaccin ou une composition comprenant un antigène en absence dudit activateur de cellules γδT.

**9.** Composition selon l'une quelconque des revendications 1 à 8 comprenant entre environ 50 μg et environ 100 mg dudit activateur de cellules γδT.

**10.** Composition selon l'une quelconque des revendications 1 à 8 comprenant entre environ 500 μg et environ 10 mg dudit activateur de cellules γδT.

**11.** Composition selon l'une quelconque des revendications 1 à 8 comprenant moins qu'environ 100 mg dudit activateur de cellules γδT.

**12.** Composition selon l'une quelconque des revendications 1 à 8 comprenant moins qu'environ 10 mg dudit activateur de cellules γδT.

**13.** Composition selon l'une quelconque des revendications 1 à 8 comprenant moins qu'environ 1 mg dudit activateur de cellules γδT.

**14.** Utilisation d'une composition vaccinale selon l'une quelconque des revendications 2-13 pour la préparation d'une composition pour améliorer la puissance d'un vaccin chez un sujet, ou pour traiter une maladie, ou pour protéger un sujet d'une maladie, plus particulièrement d'une maladie tumorale ou d'une maladie infectieuse microbienne.

**15.** Utilisation selon la revendication 14, dans laquelle ledit activateur de cellules γδT et le(s)dit(s) antigène(s) sont administrés conjointement.

**16.** Kit vaccinal comprenant un récipient adapté contenant une composition vaccinale selon l'une quelconque des revendications 2-13.

**17.** Kit vaccinal comprenant un récipient adapté contenant une composition d'adjuvant de vaccin selon l'une quelconque des revendications 1, 3-6 et 8-13 et un récipient adapté contenant un antigène ou une combinaison d'antigènes.

**18.** Utilisation selon l'une quelconque des revendications 14-17, dans laquelle ladite quantité thérapeutiquement efficace de a) un antigène ou une combinaison d'antigènes et (b) un activateur de cellules γδT est destinée à être administrée audit sujet de manière répétée après un intervalle d'au moins 1 semaine après l'administration précédente dudit antigène ou de la combinaison d'antigènes.

**19.** Utilisation selon l'une quelconque des revendications 14-17, dans laquelle ladite quantité thérapeutiquement efficace de a) un antigène ou une combinaison d'antigènes et (b) un activateur de cellules γδT est destinée à être administrée audit sujet de manière répétée après un intervalle d'au moins 2 semaines après l'administration précédente dudit antigène ou de la combinaison d'antigènes.

C-HDMAPP synthetic scheme :

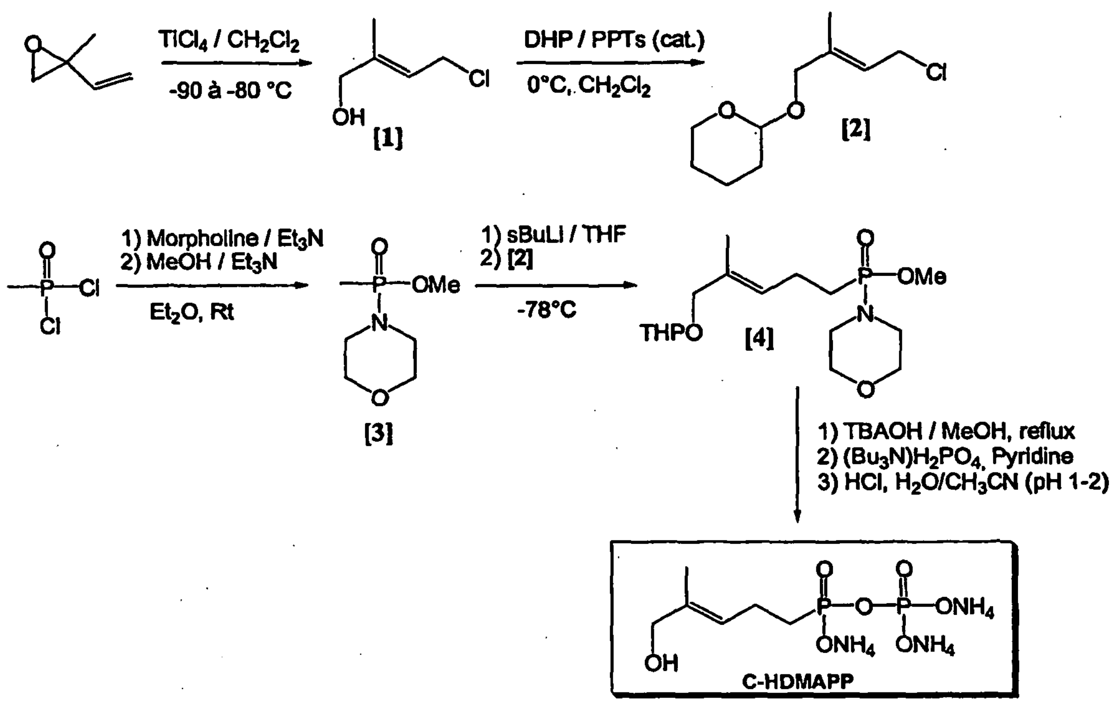


# FIGURE 1

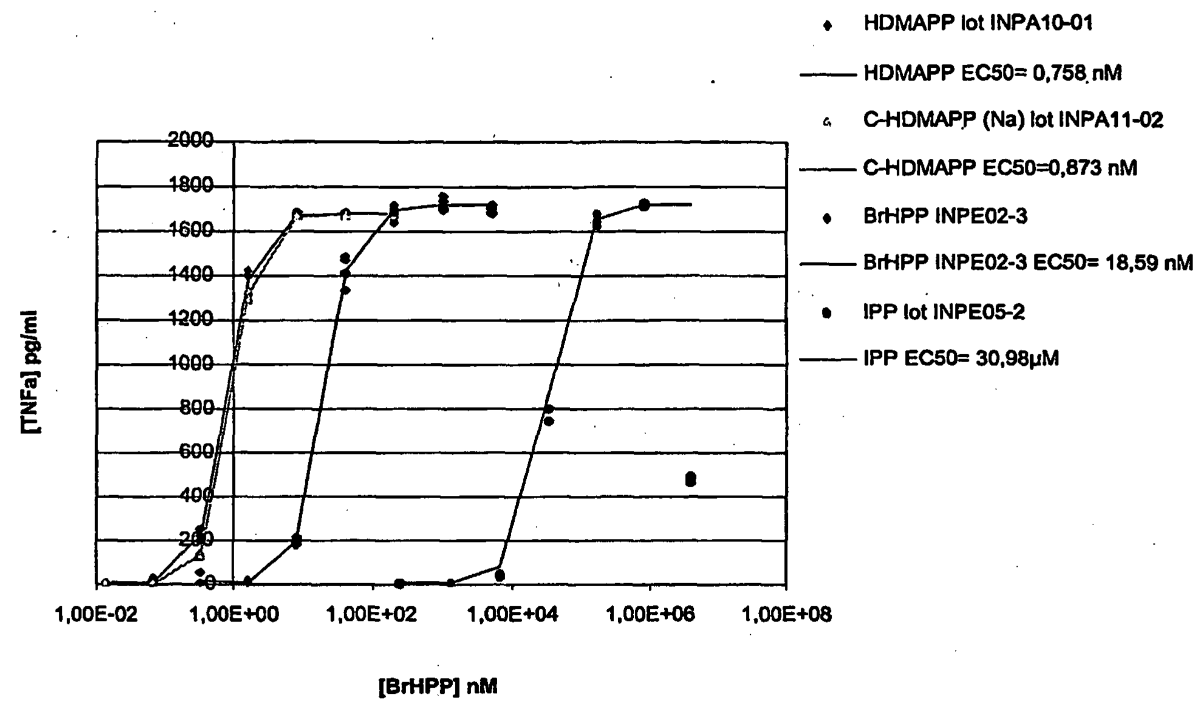
HDMAPP lot INPA10-01
HDMAPP EC50= 0,758 nM
C-HDMAPP (Na) lot INPA11-02
C-HDMAPP EC50=0,873 nM
BrHPP INPE02-3
BrHPP INPE02-3 EC50= 18,59 nM
IPP lot INPE05-2
IPP EC50= 30,98µM
[TNFa] pg/ml
2000
1800
1600
1400
1200
1000
800
600
400
200
0
1,00E-02
1,00E+00
1,00E+02
1,00E+04
1,00E+06
1,00E+08
[BrHPP] nM

# FIGURE 2

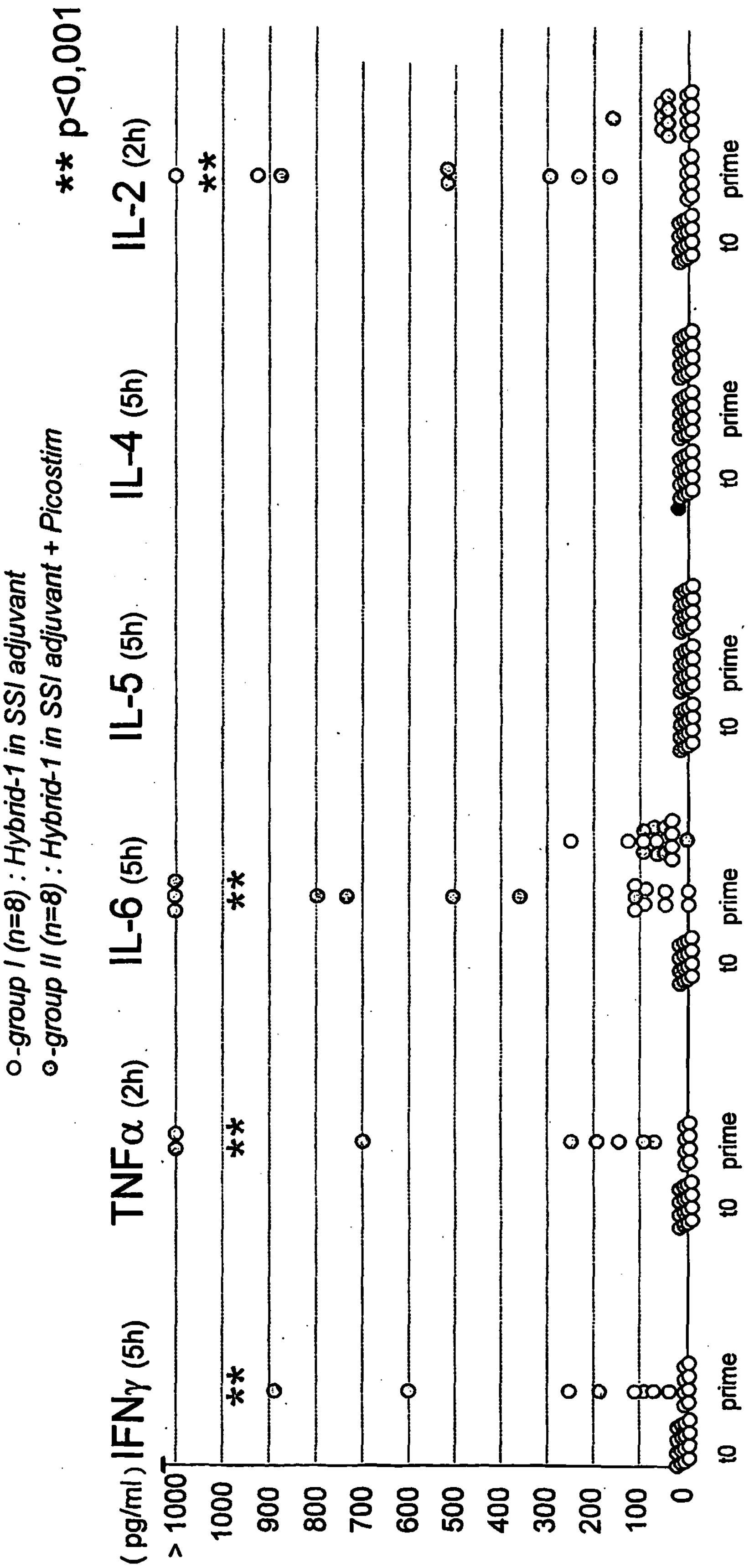
o-group I (n=8) : Hybrid-1 in SSI adjuvant
o-group II (n=8) : Hybrid-1 in SSI adjuvant + Picostim
** p<0,001
(pg/ml)
> 1000
1000
900
800
700
600
500
400
300
200
100
0
IFNγ (5h)
TNFα (2h)
IL-6 (5h)
IL-5 (5h)
IL-4 (5h)
IL-2 (2h)
**
t0
prime

FIGURE 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04050096 A
- EP 109817 B1
- WO 03050128 A
- WO 03009855 A
- WO 03009812 A
- WO 8904669 A
- WO 9011089 A
- WO 9014436 A
- US 5378814 A
- US 5171568 A
- US 5639853 A
- US 5723130 A
- US 5223254 A
- WO 9951748 A
- WO 9917741 A
- EP 366412 A
- WO 9928475 A
- WO 9006951 A
- WO 9850567 A
- WO 9520600 A
- EP 0477231 B1
- WO 9927944 A
- US 20060030546 A
- WO 05054258 A
- US 57923704 P
- US 5639653 A


### Non-patent literature cited in the description


- **BOTTINO et al.** *J Exp Med.,* 01 August 1988, vol. 168 (2), 491-505
- **DAVODEAU et al.** *Science,* 18 June 1993, vol. 260 (5115), 1800-2
- **ITO et al.** *Chest.,* 1992, vol. 102 (1), 195-7
- **MODLIN et al.** *Nature,* 1989, vol. 339 (6225), 544-8
- **WEN ; HAYDAY.** *Immunol Res.,* 1997, vol. 16 (3), 229-41
- **HAYDAY ; GENG.** *Curr Opin Immunol.,* 1997, vol. 9 (6), 884-9
- **KOBAYASHI et al.** *Cancer Immunol Immunother.,* 2001, vol. 50 (3), 115-24
- **ISMAILI et al.** *Clin Immunol.,* 2002, vol. 103 (3), 296-302
- **HINTZ et al.** *FEBS Lett.,* 2001, vol. 509 (2), 317-22
- **GUPTA et al.** *Vaccine,* 1993, vol. 1 (1), 293-306
- **BREWER et al.** *Eur. J. Immunol.,* 1996, vol. 26, 2062-2066
- **SMITH et al.** *Immunology,* 1998, vol. 93, 556562
- **ALLISON.** *Dev. Biol. Stand.,* 1998, vol. 92, 311
- **UNKELESS.** *Annu. Rev. Immunol.,* 1998, vol. 6, 251-81
- **PHILLIPS et al.** *Vaccine,* 1992, vol. 10, 151-8
- **ALLISON et al.** *Mol. Immunol.,* 1991, vol. 28, 279-84
- **WATERS et al.** *Infect Immun,* 1986, vol. 51, 816-25
- **KOS.** *Immunol. Res.,* 1998, vol. 17, 303
- **ROMAGNANI.** *Immunol. Today,* 1992, vol. 13, 379
- **BANCHEREAU ; STEINMAN.** *Nature,* 1988, vol. 392, 245
- **HOLMSKOV et al.** *Immunol. Today,* 1994, vol. 15, 67
- **ULEVITCH ; TOBIAS.** *Annu. Rev. Immunol.,* 1995, vol. 13, 437
- **TRINCHIERI.** *Annu. Rev. Immunol.,* 1995, vol. 13, 251
- **HOWARD ; O'GARRA.** *Immunol. Today,* 1992, vol. 13, 198
- **ABBAS et al.** *Nature,* 1996, vol. 383, 787
- **OKAMURA et al.** *Adv. Immunol.,* 1998, vol. 70, 281
- **MOSMANN ; SAD.** *Immunol. Today,* 1996, vol. 17, 138
- **O'GARRA.** *Immunity,* 1998, vol. 8, 275
- Fundamental Immunology. Lippincott-Raven Publ, 1999
- **CLOTHIA et al.** *J Mol. Biol.,* 1985, vol. 186, 651-663
- **HABER.** *Proc. Natl. Acad. Sci. USA,* 1985, vol. 82, 4592-4596
- **SAMBROOK ; FRITSCH ; MANIATIS.** Molecular Cloning: A Laboratory Manual. Cold Spring Harbor Laboratory Press, 1989
- DNA Cloning: A Practical Approach, Volumes. 1985, vol. I,II
- Oligonucleotide Synthesis. 1984
- Nucleic Acid Hybridization. 1985

- Transcription And Translation. 1984
- Animal Cell Culture. 1986
- Immobilized Cells And Enzymes. IRL Press, 1986
- **B. PERBAL.** *A Practical Guide To Molecular Cloning,* 1984
- Current Protocols in Molecular Biology. John Wiley & Sons, Inc, 1994
- REMINGTONS PHARMACEUTICAL SCIENCES. Mack Pub. Co, 1991
- **HOUGHTON et al.** *Hepatology,* 1991, vol. 14, 381-388
- **CHEE et al.** Cytomegaloviruses. Springer Verlag, 1990, 125-169
- **MCGEOCH et al.** *J. Gen. Virol.,* 1988, vol. 69, 1531-1574
- **BAER et al.** *Nature,* 1984, vol. 310, 207-21 1
- **DAVISON et al.** *J. Gen. Virol.,* 1986, vol. 67, 1759
- **COLLINS et al.** *Proc. Natl. Acad. Sci (USA,* 1984, vol. 81, 7683-7687
- *Biochem Biophys Acta,* 1989, vol. 67, 1007
- **RUBINS et al.** *Microbial Pathogenesis,* vol. 25, 337-342
- **COHEN et al.** *Cancer Research,* 1994, vol. 54, 1055
- **ROBBINS ; KAWAKAMI.** *Current Opinions in Immunology,* 1996, vol. 8, 628-636
- **VAN DEN EYNDE et al.** *International Journal of Clinical & Laboratory Research,* 1997
- **CORREALE et al.** *Journal of the National Cancer Institute,* vol. 89, 293
- **DAVODEAU et al.** *J. Immunology,* 1993, vol. 151 (3), 1214-1223
- **MOREAU et al.** *J. Clin. Invest.,* 1986, vol. 78, 874
- **ESPINOSA et al.** *J. Biol. Chem.,* 2001, vol. 276 (21), 18337-18344
- **WOLFF et al.** *Tetrahedron Letters,* 2002, vol. 43, 2555
- **HECHT et al.** *Tetrahedron Letters,* 2002, vol. 43, 8929
- New Trends and Developments in Vaccines. University Park Press, 1978
- **HECHT et al.** *Tetrahedron Letters,* 2002, vol. 43, 8929-8933
- **MIYASHITA et al.** *J. Org. Chem.,* 1977, vol. 42, 3772-3774
- **VALENTIJN et al.** *Synlett,* 1991, 663-664
- **PHAN ; POULTER.** *J. Org. Chem.,* 2001, vol. 66, 6705-6710
- **NOVITSKY V et al.** *J. Virol.,* 2001, vol. 75, 9210
- **DERBY et al.** *Cytokine,* 21 January 2001, vol. 13 (2), 85-90
- **NOVITSKY V et al.** Identification of human immunodeficiency virus type 1 subtype C Gag-, Tat-, Rev-, and Nef-specific ELISpot-based cytotoxic T-lymphocyte responses for AIDS vaccine design. *J. Virol.,* 2001, vol. 75, 9210
- **HAGLUND et al.** *J Virol.,* 2002, vol. 76 (15), 7506-17
- **ZHONG et al.** *Eur J Immunol.,* November 2000, vol. 30 (11), 3281-90
- **WEINRICH OLSEN A ; VAN PINXTEREN LA ; MENG OKKELS L ; BIRK RASMUSSEN P ; ANDERSEN P.** Protection of mice with a tuberculosis vaccine based on a fusion protein of Ag 85b and ESAT-6. *Infection and Immunity,* 2001, vol. 69 (5), 2773-2778
- **OLSEN AW ; WILLIAMS A ; OKKELS LM ; HATCH G ; ANDERSEN P.** Protective effect of a tuberculosis subunit vaccine based on a fusion of antigen 85b and ESAT-6 in the aerosol guinea pig model. *Infection and Immunity,* 2004, vol. 72 (10), 6148-6150